# EUROPEAN PATENT SPECIFICATION

(11) **EP 2 411 013 B1**
(45) Date of publication and mention of the grant of the patent: **19.08.2020**
(21) Application number: 10723400.7
(22) Date of filing: 25.03.2010
(51) Int. Cl.: A61K 31/5575, A61K 31/557, A61P 27/06, A61K 9/00, A61K 9/16, A61K 9/70

(54) **INTRACAMERAL SUSTAINED RELEASE DRUG DELIVERY SYSTEMS**
INTRAKAMERALE ARZNEIABGABESYSTEME MIT VERZÖGERTER FREISETZUNG
SYSTÈMES D'ADMINISTRATION INTRACAMÉRULAIRE DE MÉDICAMENT À LIBÉRATION PROLONGÉE

(30) Priority: 25.03.2009 US 41125009
(43) Date of publication of application: 01.02.2012
(73) Proprietor: ALLERGAN, INC., Irvine, CA 92612 (US)
(72) Inventor: ROBINSON, Michael R., Irvine California 92606 (US); BURKE, James A., Santa Ana California 92705 (US); LIU, Hui, Irvine California 92620 (US); ORILLA, Werhner C., Anaheim California 92804 (US); SPADA, Lon T., Walnut California 91789 (US); WHITCUP, Scott, Laguna Hill California 92653 (US); GHEBREMESKEL, Alazar N., Irvine California 92612 (US); HUGHES, Patrick M., Aliso Viejo California 92656 (US); XU, Kun, Laguna Niguel California 92677 (US); DO, Marianne M., Orange California 92866 (US)
(74) Representative: Hoffmann Eitle
(86) International application number: PCT/US2010/028584
(87) International publication number: WO 2010/111449

(56) References cited:
- WO-A1-2009/143288
- WO-A2-2008/070402
- WO-A2-2009/129187
- WO-A2-2010/062523
- US-A1- 2005 244 464
- US-A1- 2006 182 781
- US-A1- 2006 246 145
- US-A1- 2007 129 552
- US-A1- 2007 265 330
- US-A1- 2008 312 321
- KUMAR G JANORIA ET AL: "Novel approaches to retinal drug delivery" EXPERT OPINION ON DRUG DELIVERY, INFORMA HEALTHCARE, GB LNKD- DOI:10.1517/17425247.4.4.371, vol. 4, 1 July 2007 (2007-07-01), pages 371-388, XP009131521 ISSN: 1742-5247
- WOODWARD DAVID F ET AL: "Fixed-combination and emerging glaucoma therapies" EXPERT OPINION ON EMERGING DRUGS, ASHLEY PUBLICATIONS, GB LNKD- DOI:10.1517/14728214.12.2.313, vol. 12, no. 2, 1 May 2007 (2007-05-01), pages 313-327, XP009111181 ISSN: 1472-8214
- MARQUIS R E ET AL: "Management of glaucoma: Focus on pharmacological therapy" DRUGS AND AGING 2005 NZ LNKD- DOI:10.2165/00002512-200522010-00001, vol. 22, no. 1, 2005, pages 1-21, XP9136662 ISSN: 1170-229X

## Description

### BACKGROUND

The present invention relates to intraocular systems and methods for treating ocular conditions. In particular the present invention is directed to local administration of a sustained release implantcomprising an antihypertensive agent and a biodegradable polymer for use in a method for treating elevated intraocular pressure, the method comprising the step of administration to the anterior chamber (i.e. intracameral administration) and/or to anterior vitreous chamber of the eye to treat aqueous chamber elevated intraocular pressure (i.e. a hypertensive condition), as can be symptomatic of glaucoma or glaucoma risk.

The drug delivery systems of our invention can be a drug containing implant (i.e. a single, monolithic sustained release drug delivery system) or implants or a plurality of drug containing microspheres (synonymously "microparticles"). The drug delivery system can be used therapeutically to treat an ocular disease or condition such as elevated intraocular pressure and/or glaucoma. Glaucoma is a disease of the eye characterized by increased aqueous chamber intraocular pressure (IOP). Untreated glaucoma can result in blindness. Glaucoma can be primary or secondary glaucoma. Primary glaucoma in adults (congenital glaucoma) may be either open-angle or acute or chronic angle-closure. Secondary glaucoma results from pre-existing ocular diseases such as uveitis, intraocular tumor or an enlarged cataract. Various hypertensive agents have been used to lower IOP and treat glaucoma. For example, certain prostaglandins and their analogs and derivatives, such as the PGF_{2α} derivative (sometimes referred to as prostaglandin F2α analogue) latanoprost, sold under the trademark Xalatan®, have been used to treat ocular hypertension and glaucoma. Intraocular prostaglandin and prostamide implants and microspheres are disclosed by, for example U.S. patent application serial numbers 11/368,845; 11/303,462, 10/837,260, and 12/259,153. Of particular interest are Examples 1 to 5 at pages 36 to 47 of serial number 12/259,153. Also of interest is U.S. application serial number 11/952,938. Additionally, U.S. patents 5,972,326 and 5,965,152 are also of interest.

US 2005/244464, WO 2008/070402, US 2006/246145, US 2006/182781, WO 2010/062523, WO 2009/143288, WO 2009/129187, US 2007/265330, US 2007/129552 and US 2008/312321 refer to the use of known prostaglandin derivatives (like bimatoprost, latanoprost, travoprost and others) in the treatment of glaucoma.

Conventional treatment of glaucoma is by daily application of eye drops containing an anti-hypertensive drug to reduce IOP. Often patient compliance rate for regular, daily use of eyedrops is low. See eg Nordstrom et al. AJO 2005; 140: 598. Additionally, eye infection can result from improper eye dropper use. Therefore, there is a need for a long term (i.e. sustained release) treatment method for ocular hypertension that can be conveniently administered for example during a visit to the doctor's office. Hence, it would be advantageous to provide sustained release intraocular drug delivery systems (comprising implants and/or microspheres) for intraocular therapeutic use to treat elevated IOP and/or glaucoma.

### SUMMARY

The present invention meets this need and provides a sustained release intraocular drug delivery system comprising an antihypertensive agent and a biodegradable polymer for use in a method for treating elevated intraocular pressure, the method comprising the step of intracameral or anterior vitreal administration, to a patient with elevated intraocular pressure (IOP) using the above drug delivery system. The sustained release intraocular drug delivery system is in the form of an implant or microspheres which advantageously provide for extended release of one or more therapeutic anti-hypertensive agents (eg a prostaglandin or a prostamide, such as latanoprost)as shown below.

### Definitions

The following definitions are used herein.

"About" means plus or minus ten percent of the number, parameter or characteristic so qualified.

"Microsphere" and "microparticle" are used synonymously to refer to a small diameter or dimension (see below) device or element that is structured, sized, or otherwise configured to be administered intracameral. Microspheres or microparticles includes particles, micro or nanospheres, small fragments, microparticles, nanoparticles, fine powders comprising a biocompatible matrix encapsulating or incorporating a therapeutic agent. Microspheres are generally biocompatible with physiological conditions of an eye and do not cause significant adverse side effects. Microspheres made and used as disclosed herein can be administered intracameral and used safely without disrupting vision of the eye. Microspheres have a maximum dimension, such as diameter or length, less than 1 mm. For example, microparticles can have a maximum dimension less than 500 µm. Microspheres can also have a maximum dimension no greater than 200 µm, and preferably have a maximum dimension greater than 30 µm to 50 µm or to 75 microns.. An "implant" is a drug delivery device which is considerably larger than a microsphere, and whereas a plurality (i.e. hundreds or thousands)) of microspheres are administered to treat an ocular condition (such as glaucoma) usually only one to at most six implants are administered for the same purpose.

"Ocular region" or "ocular site" means any area of the eyeball, including the anterior and posterior segment of the eye, and which generally includesany functional (e.g., for vision) or structural tissues found in the eyeball, or tissues or cellular layers that partly or completely line the interior or exterior of the eyeball. Specific examples of areas of the eyeball in an ocular region include the anterior (aqueous) chamber, the posterior chamber, the vitreous cavity, the choroid, the suprachoroidal space, the conjunctiva, the subconjunctival space, the episcleral space, the intracorneal space, the epicorneal space, the sclera, the pars plana, surgically-induced avascular regions, the macula, and the retina.

An anterior ocular condition is a disease, ailment or condition which affects or which involves an anterior (i.e. front of the eye) ocular region or site, such as a periocular muscle, an eye lid or an eye ball tissue or fluid which is located anterior to the posterior wall of the lens capsule or ciliary muscles. Thus, an anterior ocular condition primarily affects or involves the conjunctiva, the cornea, the anterior chamber, the iris, the posterior chamber (behind the retina but in front of the posterior wall of the lens capsule), the lens or the lens capsule and blood vessels and nerve which vascularize or innervate an anterior ocular region or site.

Glaucoma can also be considered to be an anterior ocular condition because a clinical goal of glaucoma treatment can be to reduce a hypertension of aqueous fluid in the anterior chamber of the eye (i.e. reduce intraocular pressure).

A posterior ocular condition is a disease, ailment or condition which primarily affects or involves a posterior ocular region or site such as choroid or sclera (in a position posterior to a plane through the posterior wall of the lens capsule), vitreous, vitreous chamber, retina, optic nerve (i.e. the optic disc), and blood vessels and nerves which vascularize or innervate a posterior ocular region or site.

Glaucoma can be considered a posterior ocular condition because the therapeutic goal is to prevent the loss of or reduce the occurrence of loss of vision due to damage to or loss of retinal cells or optic nerve cells (i.e. neuroprotection).

"Biodegradable polymer" means a polymer or polymers which degrade in vivo, and wherein erosion of the polymer or polymers over time occurs concurrent with or subsequent to release of the therapeutic agent. The terms "biodegradable" and "bioerodible" are equivalent and are used interchangeably herein. A biodegradable polymer may be a homopolymer, a copolymer, or a polymer comprising more than two different polymeric units. The polymer can be a gel or hydrogel type polymer, PLA or PLGA polymer or mixtures or derivatives thereof.

"Therapeutically effective amount" means level or amount of agent needed to treat an ocular condition, or reduce or prevent ocular injury or damage without causing significant negative or adverse side effects to the eye or a region of the eye. In view of the above, a therapeutically effective amount of a therapeutic agentis an amount that is effective in reducing at least one symptom of an ocular condition.

Implants and microspheres within the scope of our invention can release an anti-hypertensive agent over a relatively long period of time, for example, for a period of time between 10 days and 120 daysafter intraocular (i.e. intracameral) administration of an anti-hypertensive agent containing implant or microspheres. Such extended release times facilitate obtaining successful treatment results. Preferably the sustained release intraocular drug delivery system is administered either intracameral (that is into the aqueous chamber [also called the anterior chamber] of the eye) or into the anterior portion of the posterior chamber (also called the vitreous chamber) of the eye.

A first embodiment of the invention is a
a sustained release implant comprising an antihypertensive agent and a biodegradable polymer for use in a method for treating elevated intraocular pressure, the method comprising the step of intracameral or anterior vitreal administration, to a patient with elevated intraocular pressure (IOP), of said sustained release implant,
wherein the sustained release implant comprises from 10 to 50 weight percent of an anti-hypertensive agent and from 50 to 90 weight percent of a biodegradable polymer,
wherein the implant releases therapeutically effective amounts of the anti-hypertensive for a period of time between 10 days and 120 days, and
wherein the anti-hypertensive agent is selected from the group consisting of Compounds A to O shown below, and their salts and esters.

A second embodiment of the invention is a plurality of sustained release biodegradable microspheres comprising an antihyperproliferative agent and a biodegradable polymer for use in a method for treating elevated intraocular pressure, the method comprising the step of intracameral or anterior vitreal administration to a patient with elevated intraocular pressure of said plurality of sustained release biodegradable microspheres having an average diameter between 30 and 60 microns,
the microspheres comprising from 10 to 30 weight percent of an anti-hypertensive agent and from 70 to 90 weight percent of a biodegradable polymer,
wherein the microspheres release therapeutically effective amounts of the anti-hypertensive agent for a period of time between 10 days and 120 days, and
wherein the anti-hypertensive agent is selected from the group consisting of Compounds A to O shown below, and their salts and esters.

A third embodiment of the invention is the microspheres for use according to embodiment 2, wherein the biodegradable polymer comprises a polylactic polyglycolic copolymer (PLGA) and/or a poly lactic acid polymer (PLA).

A fourth embodiment of the invention is the microspheres for use according to embodiment 2, wherein the microspheres comprise a high viscosity hyaluronic acid with an average molecular weight between 1 and 4 million Daltons.

Preferably, the HA is used with the plurality of microspheres formulation but not with the single implant administered. The microspheres can release the anti-hypertensive agent for at least one week after the administration step. The intraocular administration step can be carried out by injection into the sub-tenon space, such as into the anterior sub-tenon space and the pharmaceutical composition treats glaucoma by reducing baseline intraocular pressure by up to 20%, 30%, 40% or up to 50% or more.

Our invention encompasses the use of the above composition in a method for treating elevated intraocular pressure by intracameral administration to a patient with elevated intraocular pressure a plurality of sustained release biodegradable microspheres having an average diameter between 30 and 60 microns, the microspheres comprising from 10 to 50 weight percent an anti-hypertensive agent and from 50 to 90 weight percent a biodegradable polymer, wherein the microspheres release therapeutically effective amounts of the anti-hypertensive agent for a period of time between 10 days and 120 days. The biodegradable polymer can comprise a polylactic polyglycolic copolymer (PLGA) and/or a poly lactic acid polymer (PLA).

The anti-hypertensive agent can be one or more of the Compounds A to O (which are EP2 receptor agonists) (shown below), as well as their salts and

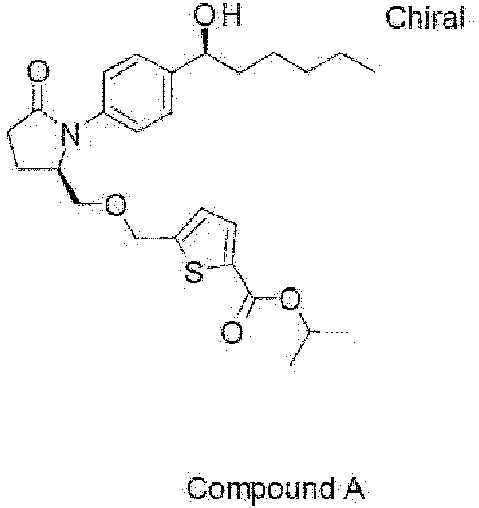

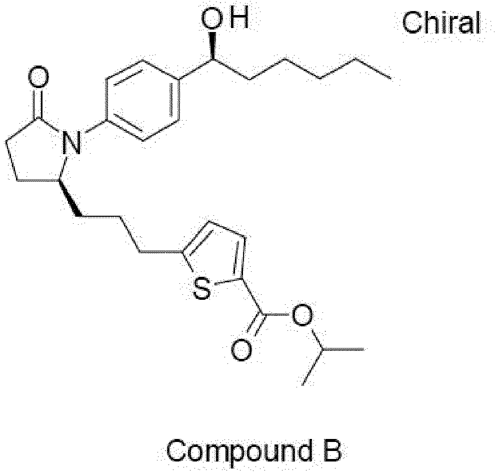

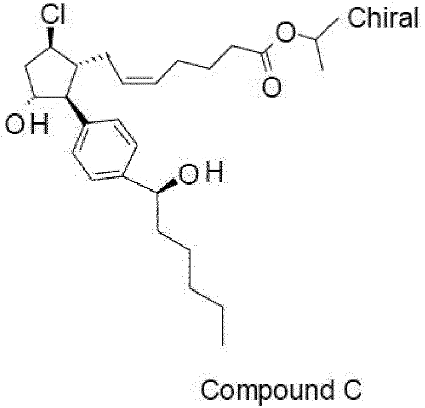

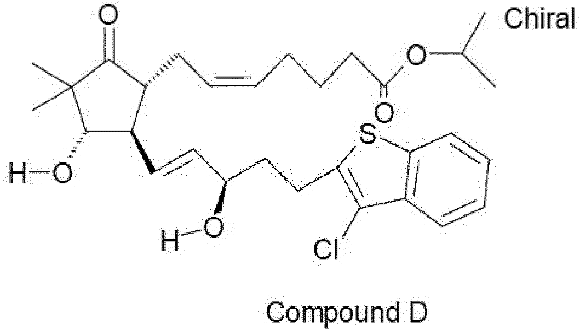

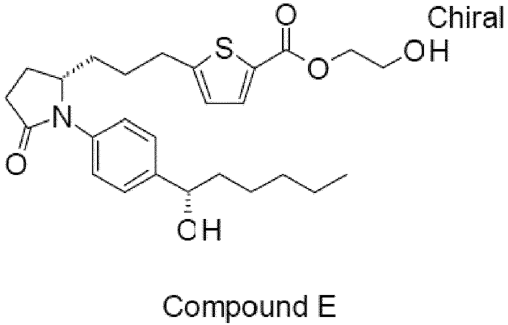

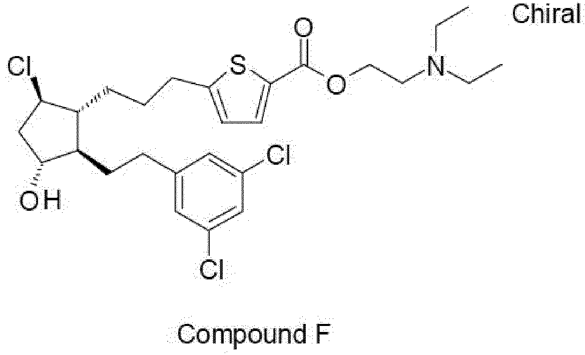

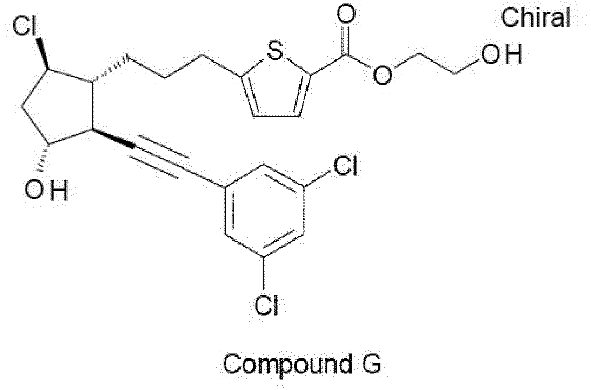

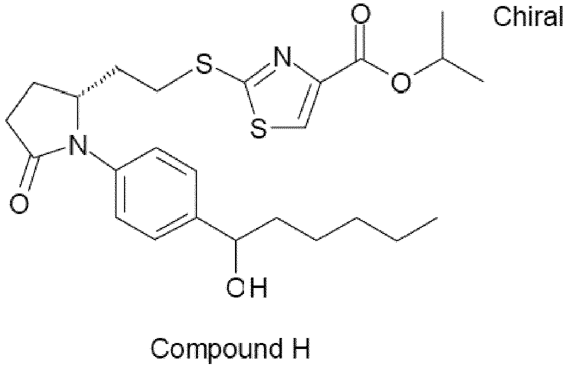

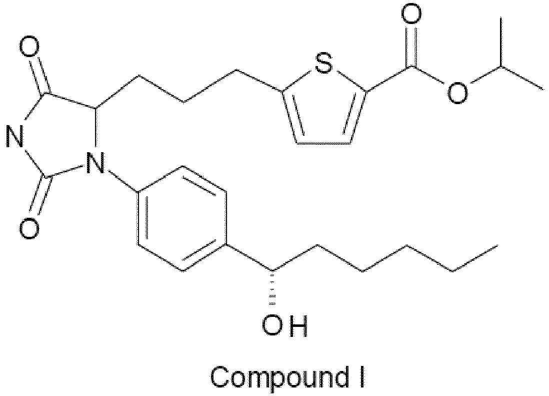

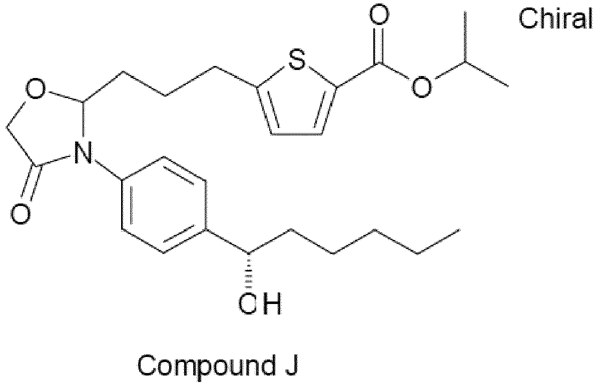

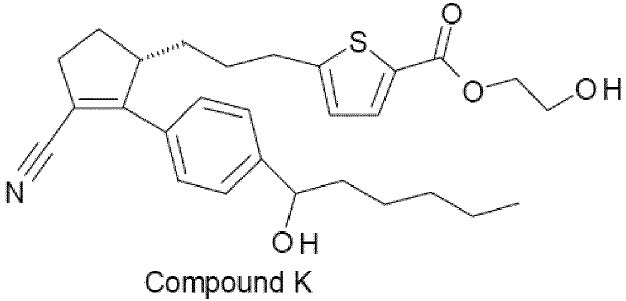

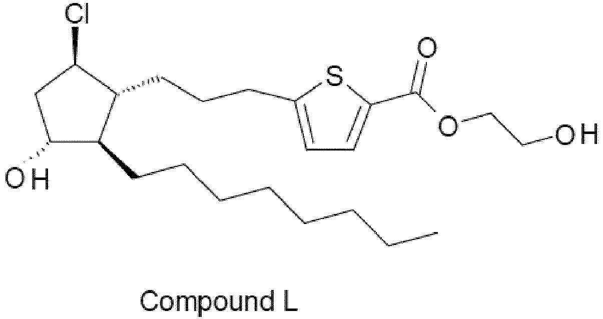

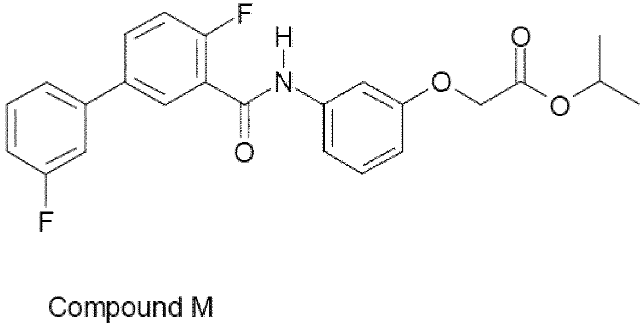

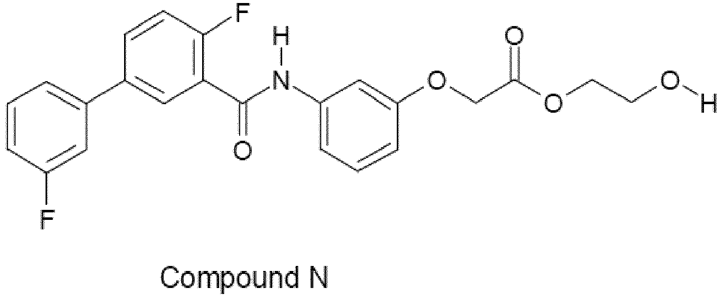

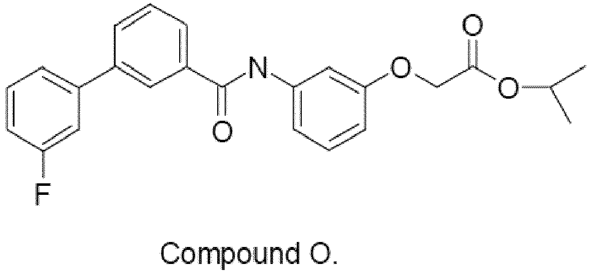

A most preferred embodiment of our invention is an intracameral placed, sustained release, rod shaped, single, monolithic (i.e. the anti-hypertensive drug is homogenously distributed [i.e. reservoir type implants are excluded from the scope of the most preferred embodiment of our invention] throughout the polymeric matrix of the implant) implant (containing a therapeutic amount of an anti-hypertensive drug) which is about 2 mm to about 4 mm long and about 0.5 mm to 2 about mm wide, implanted at the 6 o'clock or at the 12 o'clock position against the trabecular meshwork using a syringe type (i.e. 22 gauge) applicator (injector). A disc shape implant is not preferred because it will not abut well and/or will not remain in place next to the trabecular meshwork. Placement against the trabecular meshwork of a small rod-shaped implant (with the dimensions given above) takes advantage of the aqueous chamber currents and the drawing of fluid into the trabecular meshwork to hold the implant in place against the trabecular meshwork, thereby preventing the implant from floating away out of its placement position. With this most preferred embodiment there is no vision obscuration upon stable placement of the implant and no iris chafing.

Additional aspects and advantages of the present invention are set forth in the following description and claims, particularly when considered in conjunction with the accompanying drawings.

### DRAWINGS

Figure 1 is a cross-sectional representation of an area of a normal human eye anterior chamber angle showing flow direction of the aqueous humor (horizontal arrows) through the large pores in the trabeculum to the juxtacannalicular area (indicated by the vertical arrow).
Figure 2 is a schematic drawing in which the arrows show aqueous humor convection currents in the anterior chamber with microspheres indicated placed inferiorly in the anterior chamber.
Figure 3A is an external photograph of a rabbit eye in primary gaze.
Figure 3B is an image of the rabbit eye in 3A with fluorescein filters in place on a Heidelberg HRA imaging device two days after implantation of a fluorescein implant (arrow).
Figure 3C is an external photograph of the rabbit eye rotated down.
Figure 3D is an image of the rabbit eye in 3C with the HRA seven days after implantation of the fluorescein implant showing distribution (arrow) of fluorescein released from the implant.
Figure 4 is a graph showing cumulative % of latanoprost released (y axis) *in vitro* (PBS with 0.1 % triton) over time in days (x axis) from Formulation A microspheres.
Figure 5 is a graph showing cumulative % of latanoprost released (y axis) *in vitro* (PBS with 0.1% triton) over time in days (x axis) from Formulation B microspheres.
Figure 6 is a graph on the y axis percent change from baseline IOP and on the x axis time in days after drug delivery device intraocular administration. The Figure 6 results were obtained after intracameral dog (Beagle) injection of Formulation A sustained release microspheres in the left dog eye (solid line in Figure 6: "API").. The fellow (right) control eye (dashed line in Figure 6: "Control") showed no IOP reduction.
Figure 7 is a graph of percent change from subject dog eye baseline intraocular pressure (y axis) against time in days (x axis) over the 84 day period after intracameral administration of the Example 5 bimatoprost bar shaped implant ("API"), showing that an IOP drop of about 50% to 60% was maintained through the 84 day observation period. The fellow (left or "control") eye received a placebo (no bimatoprost) implant.

### DESCRIPTION

Transscleral delivery includes ocular topical (i.e. eyedrops) as well as intrascleral (i.e. subconjunctival or sub-Tenon) placement (eg by injection, insertion or implantation) placement drug administration. Our invention is based on the observation that transscleral delivery is an inefficient method for administering an anti-hypertensive agent (drug or biologic) to an aqueous chamber or vitreous chamber target tissue for the treatment of elevated intraocular pressure. We believe this to be so because of there are apparently three types of barriers hindering transscleral drug delivery - *static, dynamic,* and *metabolic* barriers. The ocular tissues that pose a physical barrier to drug diffusion (sclera, choroid-Bruch's membrane, retinal pigment epithelium) compromise the static barriers. Dynamic barriers are created by drug clearance mechanisms through blood and lymphatic vessels principally located in the conjunctiva, bulk fluid flow from anterior to posterior through the retina and clearance via the choriocapillaris and sclera, and transporter proteins of the retinal pigment epithelium. Metabolic barriers also exist in the eye and reduce drug penetration into the eye by rapid degradation of scleral administered drugs. The dynamic barriers appear to be the most important barrier to transcleral (i.e. sub-Tenon's) delivery of therapeutic agents to the front of the eye (anterior chamber) for treating ocular hypertension and glaucoma.

Our invention is based on the discovery that direct intracameral or anterior intravitreal administration of the sustained release intraocular drug delivery system as set forth herein (comprising an anti-hypertensive agent containing implants or microspheres) can be effective use to treat glaucoma, characterized by elevated intraocular pressure glaucoma by bypassing the robust scleral drug clearance mechanisms.

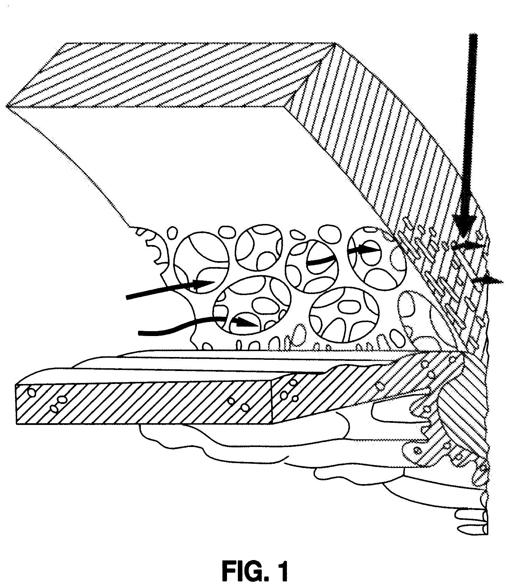

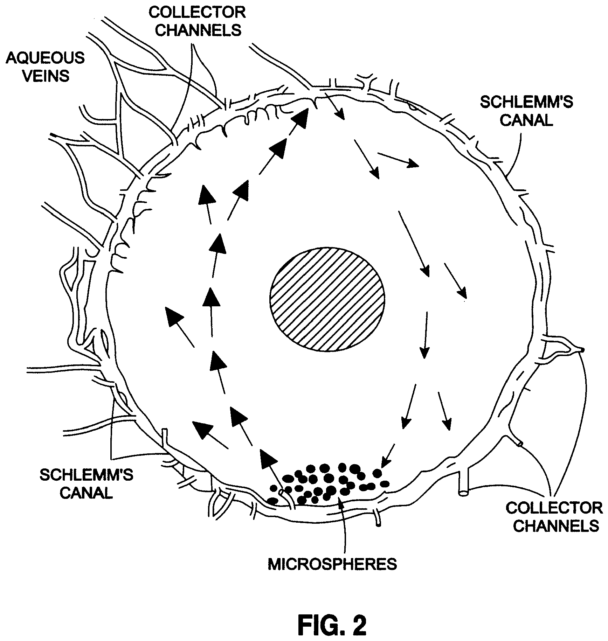

We determined existence of suitable alternative locations to deliver drugs to the front of the eye (anterior chamber) to lower the intraocular pressure (IOP) and evade the aggressive clearance of the transscleral barriers. Intracameral injections (i.e. direct injection into the anterior chamber) and anterior vitreous injections through the pars plana effectively avoid the transscleral barriers and improve the efficacy of the ocular anti-hypertensive compounds. Importantly, we discovered that intracameral drug delivery systems required development of new sustained released drug delivery system physical features, required for therapeutic efficacy because of the unique anatomy and physiology of the anterior chamber. For example, in the anterior chamber the aqueous flow rates are high and this can effectively eliminate sustained-release microspheres containing IOP lowering drugs and accelerate degradation of other polymeric delivery systems. Aqueous humor is secreted into the posterior chamber by the ciliary body, specifically by the non-pigmented epithelium of the ciliary body, through a process called ultrafiltration. It flows through the narrow cleft between the front of the lens and the back of the iris, to escape through the pupil into the anterior chamber. The aqueous humor drains 360 degrees into the trabecular meshwork that initially has pore size diameters ranging from 10 to under 30 microns in humans (see Figure 1). Figure 1 is a cross-sectional representational of the area of the eye anterior chamber angle showing the flow direction of the aqueous humor (horizontal arrows) through the larger pores (10 to less than 30 microns) in the trabeculum and gradually through to the juxtacannalicular area (indicated by the vertical arrow) where the pores reduce down to about 6 microns before entering Schlemm's canal. Aqueous humor drains through Schlemm's canal and exits the eye through 25 to 30 collector channels into the aqueous veins, and eventually into the episcleral vasculature and veins of the orbit (see Figure 2). Figure 2 is a schematic drawing in which the arrows indicate aqueous humor convection currents in the anterior chamber. Microspheres releasing anti-ocular hypertensive medication are shown placed inferiorly. Free drug eluting from the polymeric microspheres (or implant) enters the aqueous humor convection currents (arrows). The drug is then successfully dispersed throughout the anterior chamber and enters the target tissues such as the trabecular meshwork and the ciliary body region through the iris root region.

Another advantage of intracameral injection is that the anterior chamber is an immune privileged site in the body and less likely to react to foreign material, such as polymeric drug delivery systems. This is not the case in the sub-Tenon's space where the inflammatory reactions to foreign materials are common. In addition to the anterior chamber containing immunoregulatory factors that confers immune privilege, particles with diameters greater than 30 microns are less immunogenic and have a lower propensity towards causing ocular inflammation. Resident macrophages in the eye are the first line of defense with foreign bodies or infectious agents; however, particles larger than 30 microns are difficult to phagocytose. Therefore, particles larger than 30 microns are less prone to macrophage activation and the inflammatory cascade that follows.

We found that the efficiency of delivering drug to the aqueous humor with a polymeric release system is much greater with an intracameral location vs. sub-Tenon's application. Thus, less than 1% of drug delivered in the sub-Tenon's space will enter the aqueous humor whereas 100% of the drug released from an intracameral system will enter the aqueous humor. Therefore, it is expected that there will be lower drug loads required for effective intracameral drug delivery systems compared with sub-Tenon's and consequently, less systemic drug exposure. In addition, there will be less exposure of the conjunctiva to the active pharmaceutical ingredient, and less propensity towards developing conjunctival hyperemia when delivering drugs such as the prostaglandin analogues. Lastly, the drug will enter the conjunctival/episcleral blood vessel directly following an intracameral injections via the aqueous veins. This may minimize conjunctival hyperemia with prostaglandin analogues compared with a sub-Tenon's injection where numerous vessels are at risk of dilation with a high concentration of drug present diffusely in the extravascular space of the conjunctiva. Direct injections into the eye also obviate the need for preservatives, which when used in topical drops, can irritate the ocular surface.

We have developed implants and microspheres which can release drug loads over various time periods. These implants or microspheres, which when inserted intracameral or into the anterior vitreous therapeutic provide therapeutic levels of an anti-hypertensive agent for extended periods of time (e.g., for about 1 week up to about one year). Additionally, we have developed novel methods for making implants and microspheres. The anti-hypertensive agent of the present implants and microspheres is from 1 % to 50% by weight of the microspheres. More preferably, the anti-hypertensive agent is from 5% to 30% by weight of the implant or microspheres. In a preferred embodiment, the anti-hypertensive agent comprises 15% by weight of the microsphere (e.g., 5%-30 weight %). In another embodiment, the anti-hypertensive agent comprises 40% by weight of the microspheres.

Suitable polymeric materials or compositions for use in the implant or microspheres include those materials which are compatible, that is biocompatible, with the eye so as to cause no substantial interference with the functioning or physiology of the eye. Such materials preferably are at least partially and more preferably substantially completely biodegradable or bioerodible.

Examples of useful polymeric materials include, without limitation, such materials derived from and/or including organic esters and organic ethers, which when degraded result in physiologically acceptable degradation products, including the monomers. Also, polymeric materials derived from and/or including, anhydrides, amides, orthoesters and the like, by themselves or in combination with other monomers, may also find use. The polymeric materials may be addition or condensation polymers, advantageously condensation polymers. The polymeric materials may be cross-linked or non-cross-linked, for example not more than lightly cross-linked, such as less than 5%, or less than 1 % of the polymeric material being cross-linked. For the most part, besides carbon and hydrogen, the polymers will include at least one of oxygen and nitrogen, advantageously oxygen. The oxygen may be present as oxy, e.g. hydroxy or ether, carbonyl, e.g. non-oxo-carbonyl, such as carboxylic acid ester, and the like. The nitrogen may be present as amide, cyano and amino. The polymers set forth in Heller, Biodegradable Polymers in Controlled Drug Delivery, In: CRC Critical Reviews in Therapeutic Drug Carrier Systems, Vol. 1, CRC Press, Boca Raton, FL 1987, pp 39-90, which describes encapsulation for controlled drug delivery, may find use in the present microspheres.

Of additional interest are polymers of hydroxyaliphatic carboxylic acids, either homopolymers or copolymers, and polysaccharides. Polyesters of interest include polymers of D-lactic acid, L-lactic acid, racemic lactic acid, glycolic acid, polycaprolactone, and combinations thereof. Generally, by employing the L-lactate or D-lactate, a slowly eroding polymer or polymeric material is achieved, while erosion is substantially enhanced with the lactate racemate. Among the useful polysaccharides are, without limitation, calcium alginate, and functionalized celluloses, particularly carboxymethylcellulose esters characterized by being water insoluble, a molecular weight of about 5 kD to 500 kD, for example.

Other polymers of interest include, without limitation, polyvinyl alcohol, polyesters, polyethers and combinations thereof which are biocompatible and may be biodegradable and/or bioerodible. Some preferred characteristics of the polymers or polymeric materials for use in the present invention may include biocompatibility, compatibility with the selected therapeutic agent, ease of use of the polymer in making the drug delivery systems of the present invention, a half-life in the physiological environment of at least about 6 hours, preferably greater than about one day, and water insolubility.

The biodegradable polymeric materials which are included to form the matrix are desirably subject to enzymatic or hydrolytic instability. Water soluble polymers may be cross-linked with hydrolytic or biodegradable unstable crosslinks to provide useful water insoluble polymers. The degree of stability can be varied widely, depending upon the choice of monomer, whether a homopolymer or copolymer is employed, employing mixtures of polymers, and whether the polymer includes terminal acid groups.

Equally important to controlling the biodegradation of the polymer and hence the extended release profile of the implant is the relative average molecular weight of the polymeric composition employed in the implants or microspheres. Different molecular weights of the same or different polymeric compositions may be included in the microspheres to modulate the release profile.

In some implants and microspheres, copolymers of glycolic acid and lactic acid are used, where the rate of biodegradation is controlled by the ratio of glycolic acid to lactic acid. The most rapidly degraded copolymer has roughly equal amounts of glycolic acid and lactic acid. Homopolymers, or copolymers having ratios other than equal, are more resistant to degradation. The ratio of glycolic acid to lactic acid will also affect the brittleness of the microspheres. The percentage of polylactic acid in the polylactic acid polyglycolic acid (PLGA) copolymer can be 0-100%, preferably 15-85%, more preferably 35-65%. In some implants, a 50/50 PLGA copolymer is used.

The implants and microspheres can be monolithic, i.e. having the active agent or agents homogenously distributed through the polymeric matrix, or encapsulated, where a reservoir of active agent is encapsulated by the polymeric matrix. Due to ease of manufacture, monolithic implants are usually preferred over encapsulated forms. However, the greater control afforded by the encapsulated microspheres may be of benefit in some circumstances, where the therapeutic level of the drug falls within a narrow window. In addition, the therapeutic component, including the latanoprost component, may be distributed in a non-homogenous pattern in the matrix. For example, the microspheres may include a portion that has a greater concentration of the latanoprost relative to a second portion of the microspheres.

The microspheres disclosed herein may have a size of between about 5µm and 1 mm, or between 10 µm and 0.8 mm for administration with a needle. For needle-injected microspheres, the microspheres may have any appropriate dimensions so long as the longest dimension of the microsphere permits the microsphere to move through a needle. This is generally not a problem in the administration of microspheres.

The total weight of implant or microsphere in a single dosage an optimal amount, depending on the volume of the anterior chamber and the activity or solubility of the active agent. Most often, the dose is usually 0.1 mg to 200 mg of implant or microspheres per dose. For example, a single intracameral injection may contain 1 mg, 3 mg, or 5 mg, or 8 mg, or 10 mg, or 100 mg or 150 mg, or 175 mg, or 200 mg of microspheres, including the incorporated therapeutic component.

The implant or microspheres may be of any particulate geometry including micro and nanospheres, micro and nanoparticles, spheres, powders, fragments and the like. The upper limit for the microsphere size will be determined by factors such as toleration for the implant, size limitations on insertion, desired rate of release, ease of handling, etc. Spheres may be in the range of 0.5 µm to 4 mm in diameter, with comparable volumes for other shaped particles.

The proportions of the anti-hypertensive agent, polymer, and any other modifiers may be empirically determined by formulating several microsphere batches with varying average proportions. A USP approved method for dissolution or release test can be used to measure the rate of release (USP 23; NF 18 (1995) pp. 1790-1798). For example, using the infinite sink method, a weighed sample of the microspheres is added to a measured volume of a solution containing 0.9% NaCl in water, where the solution volume will be such that the drug concentration is after release is less than 5% of saturation. The mixture is maintained at 37°C and stirred slowly to maintain the microspheres in suspension. The appearance of the dissolved drug as a function of time may be followed by various methods known in the art, such as spectrophotometrically, HPLC, mass spectroscopy, etc. until the absorbance becomes constant or until greater than 90% of the drug has been released.

In addition to the therapeutic component, the implants and microspheres disclosed herein may include or may be provided in compositions that include effective amounts of buffering agentsand/or preservatives. Suitable water-soluble buffering agents include, without limitation, alkali and alkaline earth carbonates, phosphates, bicarbonates, citrates, borates, acetates, succinates and the like, such as sodium phosphate, citrate, borate, acetate, bicarbonate and carbonate. These agents advantageously present in amounts sufficient to maintain a pH of the system of between 2 to 9 and more preferably between 4 to 8. As such the buffering agent may be as much as 5% by weight of the total implant. Suitable water-soluble preservatives include sodium bisulfite, sodium bisulfate, sodium thiosulfate, ascorbate, benzalkonium chloride, chlorobutanol, thimerosal, phenylmercuric acetate, phenylmercuric borate, phenylmercuric nitrate, parabens, methylparaben, polyvinyl alcohol, benzyl alcohol, phenylethanol and mixtures thereof. These agents may be present in amounts of from 0.001 % to 5% by weight and preferably 0.01 % to 2% by weight. In at least one of the present microspheres, a benzylalkonium chloride preservative is provided in the implant, such as when the latanoprost consists essentially of bimatoprost

Various techniques may be employed to produce the implants and/or microspheres described herein. Useful techniques includeself-emulsification methods, super critical fluid methods, solvent evaporation methods, phase separation methods, spray drying methods, grinding methods, interfacial methods, molding methods, injection molding methods and combinations thereof.

As discussed herein, the polymeric component recited in the present method comprises a biodegradable polymer or biodegradable copolymer. In at least one embodiment, the polymeric component comprises a poly (lactide-co-glycolide) PLGA copolymer. In a further embodiment, the PLGA copolymer has a lactide/glycolide ratio of 75/25. In a still further embodiment, the PLGA copolymer has at least one of a molecular weight of about 63 kilodaltons and an inherent viscosity of about 0.6 dL/g.

In addition, the present population of microparticles may have a maximum particle diameter less than 200 µm. In certain embodiments, the population of microparticles has an average or mean particle diameter less than 50 µm. In further embodiments, the population of microparticles has a mean particle diameter from 30 µm to 60 µm.

The anti-hypertensive agent containing implants and microspheres disclosed herein can be used to treat glaucoma. A pharmaceutical composition (such as an implant or microspheres) within the scope of our invention can be formulated with a high viscosity, polymeric gel to reduce dispersion of the composition upon intraocular injection. Preferably, the gel has a high shear characteristic, meaning that the gel can be injected into an intraocular site through a 25-30 gauge needle, and more preferably through a 27-30 gauge needle. A suitable gel for this purpose can be a hydrogel or a colloidal gel formed as a dispersion in water or other aqueous medium. Examples of suitable gels include synthetic polymers such as polyhydroxy ethyl methacrylate, and chemically or physically crosslinked polyvinyl alcohol, polyacrylamide, poly(N-vinyl pyrolidone), polyethylene oxide, and hydrolysed polyacrylonitrile. Examples of suitable hydrogels which are organic polymers include covalent or ionically crosslinked polysaccharide-based hydrogels such as the polyvalent metal salts of alginate, pectin, carboxymethyl cellulose, heparin, hyaluronate (i.e. polymeric hyaluronic acid) and hydrogels from chitin, chitosan, pullulan, gellan, xanthan and hydroxypropylmethylcellulose. Commercially available dermal fillers (such as Hylafrom®, Restylane®, Sculptura™ and Radiesse) can be used as the high viscosity gel in embodiments of our pharmaceutical composition.

Hyaluronic acid ("HA") is a polysaccharide made by various body tissues. U.S. patent 5,166,331 discusses purification of different fractions of hyaluronic acid for use as a substitute for intraocular fluids and as a topical ophthalmic drug carrier. Other U.S. patent applications which discuss ocular uses of hyaluronic acid include serial numbers 11/859,627; 11/952,927;10/966,764; 11/741,366; and 11/039,192 The pharmaceutical compositions within the scope of our invention preferably comprise a high viscosity hyaluronic acid with an average molecular weight between 1 and 4 million Daltons, and more preferably with an average molecular weight between 2 and 3 million Daltons, and most preferably with an average molecular weight of (± 10%) 2 million Daltons.

Dry uncrosslinked HA material comprises fibers or powder of commercially available HA, for example, fibers or powder of sodium hyaluronate (NaHA). The HA may be bacterial-sourced sodium hyaluronate, animal derived sodium hyaluronate or a combination thereof. In some embodiments, the dry HA material is a combination of raw materials including HA and at least one other polysaccharide, for example, glycosaminoglycan (GAG). In our invention the HA used comprises or consists of high molecular weight HA. That is, nearly 100% of the HA material in the present compositions is a high molecular weight HA. High molecular weight HA means HA with a molecular weight of at least 1.0 million Daltons (mw ≥ 10⁶ Da) to 4.0 million Da (mw ≤ 4 X 10⁶ Da). For example, the high molecular weight HA in the present compositions may have a molecular weight of 2.0 million Da (mw 2 X 10⁶ Da). In another example, the high molecular weight HA may have a molecular weight of 2.8 million Da (mw 2.8 x 10⁶ Da).

In an embodiment of our invention, dry or raw HA material (in this specific example, NaHA) having a desired high/low molecular weight ratio is cleaned and purified. These steps generally involved hydrating the dry HA fibers or powder in the desired high/low molecular weight ratio, for example, using pure water, and filtering the material to remove large foreign matters and/or other impurities. The filtered, hydrated material is then dried and purified. The high and low molecular weight NaHA may be cleaned and purified separately, or may be mixed together, for example, in the desired ratio, just prior to crosslinking. At this stage in the process, the pure, dried NaHA fibers are hydrated in an alkaline solution to produce an uncrosslinked NaHA alkaline gel. Any suitable alkaline solution may be used to hydrate the NaHA in this step, for example to an aqueous solution containing NaOH. The resulting alkaline gel will have a pH above 7.5, for example, a pH above 8, for example, a pH above 9, for example, a pH above 10, for example, a pH above 12, for example, a pH above 13. In this specific example, the next step in the manufacturing process comprises the step of crosslinking the hydrated, alkaline NaHA gel with a suitable crosslinking agent, for example, BDDE.

The step of crosslinking may be carried out using means known to those of skill in the art. Those skilled in the art appreciate how to optimize the conditions of crosslinking according to the nature of the HA, and how to carry out the crosslinking to an optimized degree. In some embodiments of the present invention, the degree of crosslinking is at least 2% to 20%, for example, is 4% to 12%, wherein the degree of crosslinking is defined as the percent weight ratio of the crosslinking agent to HA-monomeric units in the composition. The hydrated crosslinked, HA gel may be neutralized by adding an aqueous solution containing HCI. The gel is then swelled in a phosphate buffered saline solution for a sufficient time and at a low temperature.

In certain embodiments, the resulting swollen gel (HA) is a cohesive gel having substantially no visible distinct particles, for example, substantially no visibly distinct particles when viewed with the naked eye. In some embodiments, the gel has substantially no visibly distinct particles under a magnification of less than 35X. The gel ((HA) is now purified by conventional means for example, dialysis or alcohol precipitation, to recover the crosslinked material, to stabilize the pH of the material and remove any unreacted crosslinking agent. Additional water or slightly alkaline aqueous solution can be added to bring the concentration of the NaHA in the composition to a desired concentration. In some embodiments, the concentration of NaHA in the composition is in a range between 10 mg/ml to 30 mg/ml.

Implants within the scope of our invention can be administered using any suitable intraocular injection device including the applicators (injectors) shown in U.S. patent applications 11/455,392; 11/552,835; 11/552,630, and 12/355,709.

Embodiments of our invention can be sustained release biodegradable microspheres or implants. A preferred embodiment of our invention is a PLA and/or PLGA implant containing an anti-hypertensive agent because we have determined that implants of such composition result in significantly less inflammatory (i.e. less corneal hyperemia) upon intracameral or anterior vitreal administration. An embodiment of our invention can comprise a drug delivery system with a plurality of anti-hypertensive agents contained in different segments of the same implant or in different implants administered at the same time. For example, one segment (i.e. one implant) can contain a muscarinic anti-hypertensive agent, a second segment (i.e. a second implant) can contain a anti-hypertensive prostaglandin and third segment (i.e. a third implant) can contain an anti-hypertensive beta blocker. Multiple implants ("segments") can be injected simultaneously, for example, one implant with an anti-hypertensive agent to enhance aqueous outflow through the trabecular meshwork (e.g. a muscarinic agent), a second implant can be used to enhance uveoscleral flow (e.g. a hypotensive lipid), and a third implant can reduce aqueous humor production (e.g. a beta blocker). Multiple hypotensive agents with different mechanisms of action can be more effective at lowering IOP than monotherapy, that is use of a single type of an anti-hypertensive agent. Multiple segments (implants) have the advantage of permitting lower doses of each separate anti-hypertensive agent used than the dose necessary with monotherapy, thereby reducing the side effects of each anti-hypertensive agent used. A separate and additional segment, containing for example a neuroprotective or neuroenhancing compound, can also be delivered with other segments containing anti-hypertensive agents.

When using multiple segments (i.e. a plurality of implants administered), each segment is preferably has a length no greater than about 2 mm. Preferably, the total umber of segments administered in the same a 22 to 25G diameter needle bore is about four. With a 27G diameter needle total segments length within the needle bore or lumen can be up to about 12 mm.

We determined that the trabecular meshwork (TM) has a detectable fluid uptake or suction action upon aqueous chamber fluid. This TM fluid uptake causes microspheres (MS) with a diameter of less than 30 microns to be drawn into the TM as we determined by gonioscopy imaging.

We also determined that the fluid uptake action of the TM can be exploited to keep MS or implants that have an appropriate geometry from floating around the anterior chamber causing visual obscuration. Gravity brings these implants down to the 6 o'clock position and we noted the implants or MS are very stable (relatively immobile) in this position. Implants that can be intraocular administered by a 22G to 30G diameter needle with lengths totaling no more than about 6 to 8 mm (all segments included) are most preferred to take advantage of the TM fluid uptake mechanism with resulting intraocular implant immobility and no visual obscuration. Thus, despite being firmly in the 6 o'clock position in the anterior chamber due to TM fluid uptake effect, the implants can have release rates that exceed the TM clearance rate and this allows anti-hypertensive agent released by the implants to rapidly fill the anterior chamber and distribute well into the target tissues along a 360 degrees distribution pattern. Our examination of the implants in the angle of the anterior chamber with gonioscopy showed that the there was no encapsulation of nor inflammatory tissue in the vicinity of the implants.

### EXAMPLES

The following examples set forth embodiments of our invention.

### Example 1

### Determination of Anterior Chamber Convection Currents

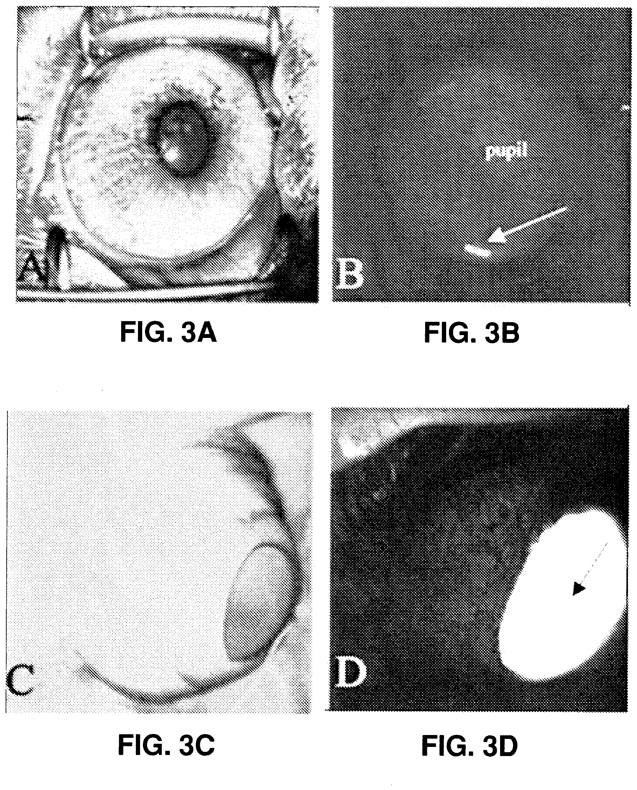

We determined that in the anterior chamber of the eye there are vertical upwards convection currents flowing from the 6 o'clock position to the 12 o'clock position driven by the higher temperatures of the aqueous humor in contact with the iris. We also determined that in the anterior chamber there are downward convection currents of aqueous flow proceeding from the 12 o'clock position to the 6 o'clock position driven by the cooler temperatures of aqueous humor adjacent to the corneal endothelium. See Figure 2. We hypothesized that these aqueous humor currents can effectively carry anti-hypertensive drugs throughout and around the anterior chamber in a 360 degrees distribution pattern if the delivery system releases drug directly into the aqueous humor and we demonstrated the effectiveness of the convection currents distributing a surrogate drug in the anterior chamber with imaging studies of release from a sustained-release implant placed in the anterior chamber. See Figure 3.

Additionally, we made microspheres with diameters greater than 30 microns and with sufficient density to settle into the inferior angle of the anterior chamber following intracameral injection of the microspheres. Importantly, the greater than 30 micron diameter of the microspheres is such that the microspheres are not either cleared through or embedded in the trabecular meshwork, thereby ensuring that free drug is released directly into the aqueous currents to effectively distribute drug to the angle along a 360 degrees distribution pattern. Free drug can transit through the trabecular meshwork and the iris root into the ciliary body region. To accelerate settling of microsphere formulations to the 6 o'clock position, an uncrosslinked or cross-linked hydrogel, such as a hyaluronic acid or a methylcellulose compound can be added in a 0.2 % to 4 % concentration to the microspheres, as a carrier. The addition of the gel can facilitate passage of microspheres with diameters greater than 30 microns through small gauge (e.g. 27 to 30G) needles and permits use in pre-filled syringes. Alternative delivery systems, such as solid implants with bioerodible polymers can also be used since they will settle at the 6 o'clock position following injection into the anterior chamber (see Figure 3B).

Thus, Figure 3 presents evidence of aqueous humor drug delivery (after intracameral placement of an implant at the 6 o'clock position) via convection currents visualized using a Heidelberg HRA imaging device. Figure 3A is an external photograph of a rabbit eye in primary gaze. Figure 3B is an image of the rabbit eye in 3A with fluorescein filters in place on the HRA. In Figure 3B the rabbit is 2 days post-implantation of a sustained-release fluorescein implant in the anterior chamber and it can be seen that the implant has settled at the 6 o'clock position (arrow). Figure 3C is an external photograph of the same rabbit eye rotated down.

Figure 3D is an image of the rabbit eye in 3C with the HRA. In Figure 3D the rabbit is 7 days post-implantation of a sustained-release fluorescein implant in the anterior chamber that has settled at the 6 o'clock position as shown in 3B. Note that with the convection currents, free fluorescein released from the implant has become evenly distributed throughout the anterior chamber (arrow) and will thereby have 360 degree exposure to the trabecular meshwork and ciliary body, the target tissues for anti-hypertensive treatment.

We also determined that microspheres or other sustained-release delivery systems that have a lower density than the aqueous humor can have therapeutic utility because they will float up and settle superiorly in the 12 o'clock position. Here the drug delivery system can release drug into the convection currents and this is a suitable alternative to delivery systems that are located at the 6 o'clock position in order to distribute free drug to the angle 360 degrees. Thus, we took time lapsed images of a drug surrogate injected at 12 o'clock position and demonstrated presence of anterior chamber convection currents which distributed the drug surrogate throughout (homogenous drug exposure over 360 degrees) the anterior chamber within 20 minutes.

As set forth below we developed a technique where the PVA stabilizer used in the manufacturing process was washed with water 5 times to strip the PVA component off the microspheres. This chemical modification allowed the microspheres to float up to the 12 o'clock position in the anterior chamber because microsphere surfaces become very hydrophobic after losing hydrophilic PVA and water cannot effectively wet particle surfaces. It is critical for the drug delivery system to rapidly settle inferiorly or superiorly to clear the visual axis of any obstruction.

Unexpectedly, pharmacokinetic studies examining drug tissue levels in the ciliary body demonstrated high levels following injection of sustained-release implants into the anterior vitreous region. It has been previously thought that most drugs injected into the vitreous cavity would be diffuse and/or be directed by various mechanisms to the back of the eye and eliminated through the retina and choroid. We carried out imaging and pharmacologic studies and placement of delivery systems in the anterior vitreous base and determined that delivery of anti-hypertensive drugs to the ciliary body can thereby be achieved with resultant lower IOP. These imaging studies demonstrated that drug placed into the anterior vitreous base can access the aqueous humor in the posterior chamber and rapidly disperse drug 360 degrees in both animal and human eyes. Drug delivery systems, such as microspheres and implants, can be routinely placed into the anterior vitreous using standard surgical procedures. The MRI imaging studies were with porcine eye following injection of a drug surrogate into the anterior vitreous. The drug passed rapidly into the posterior chamber and was distributed around the ciliary body in a 360 degrees pattern. Additionally, we carried out MRI imaging studies of a human eye following injection of a drug surrogate in the anterior vitreous and demonstrated that the drug passed rapidly into the posterior and anterior chamber, showing that vitreous injections can deliver drugs to the aqueous humor.

### Example 2

### Development of Sustained Release Microspheres

### Introduction

In this Example we made and evaluated various hypertensive drug containing microspheres for use to treat glaucoma. Thus, we developed sustained release microspheres for treatment of ocular hypertension. The microspheres we made can provide from 3 months to 6 months of IOP reduction (as a monotherapy, that is without the need for supplemental anti-hypertensive drug containing eye drops) with very reduced corneal hyperemia (as compared to sub-tenon administration of the same microspheres or implant). The microspheres contain at least 10 weight % hypertensive drug load and have a mean diameter of greater than 30 µm, as we determined that use of microspheres with a diameter greater than 30 µm reduces eye hyperemia after intracameral administration of the microspheres.

The microsphere manufacturing process was started with a solvent evaporation process using dichloromethane as a solvent and SDS surfactant. However, when latanoprost was incorporated, the process had numerous problems with very low yields, much smaller particle sizes, and poor drug entrap efficiencies. Therefore, we developed process improvements by changing both the solvent and the surfactant. Eventually, we finalized the process with ethyl acetate as the solvent and 1 % poly vinyl alcohol (PVA) as stabilizer. Also, we were able to obtain hypertensive drug loading as high as 19 wt %. The microsphere diameters were maintained above 30 µm and can be made up to 65 µm if reduced shear rates were used. Microsphere diameter and diameter distribution were determined using a Malvern Mastersizer 2000 instrument. Each sample was analyzed by average of 5 readings. Microsphere fractionations were also practiced by filtering through sieves to maintain minimum size cutoff. Many different PLA and PLGA polymers and polymer blends were screened to obtain a family of release profiles and to select candidates for *in vivo* microsphere administration. The *in vitro* release rate of the formulations studied ranged from 17 to 88 µg/day.

Extensive morphological studies were performed on the microspheres made. Thus, microsphere surfaces were examined by SEM (using a Zeiss EVO 40 instrument), and the drug distribution inside the particle were determined by freeze facture SEM. Surface and internal morphology was examined using SEM freeze-dried microspheres which were dusted over double-sided adhesive graphite tape with the other side applied to an aluminum stub. Excess samples were removed and stub sputter coated with a 5-10 nm gold layer. Internal microsphere morphology was observed following microsphere freeze fracture carried out by applying monolayer microspheres on carbon tape, covered with another carbon tape on stub, and this sandwich structure was then submerged into liquid nitrogen for 10 seconds. The sandwiched monolayer was broken up to result into fractured microspheres.

Samples before and after drug release were compared, and drug loaded samples were also compared with placebos. They exhibit strikingly different morphologies, and revealed close relationships among polymer properties, morphologies, and release behaviors in all twenty three different microsphere formulations we made. The two microsphere Formulation A and Formulation B were evaluated *in vivo.* We determined that these sustained release microsphere formulations A and B can release of anti-hypertensive agent over a several month period and that they the microspheres can be administrated by intraocular injection on an outpatient basis.

### Formulation A Microspheres (reference)

Initially, we found that introduction of latanoprost into the polymer matrix significantly reduced the cohesion and resulted in small microparticle diameters. Additionally, poor drug entrap efficiencies (low wt % drug load) were attributed to much increased drug solubility in water due to SDS and long slow DCM evaporation process. DCM is not water miscible, and its evaporation process takes quite long time during which latanoprost has plenty of time to diffuse into water phase. We carried out experiments to decrease latanoprost aqueous solubility and to change the stabilizer used (from SDS to polyvinyl alcohol). Another process improvement was to gradually add more water miscible solvent, such as acertonitrile or ethyl acetate. This expedited particulate drying process. The final process used was a solvent extraction process.

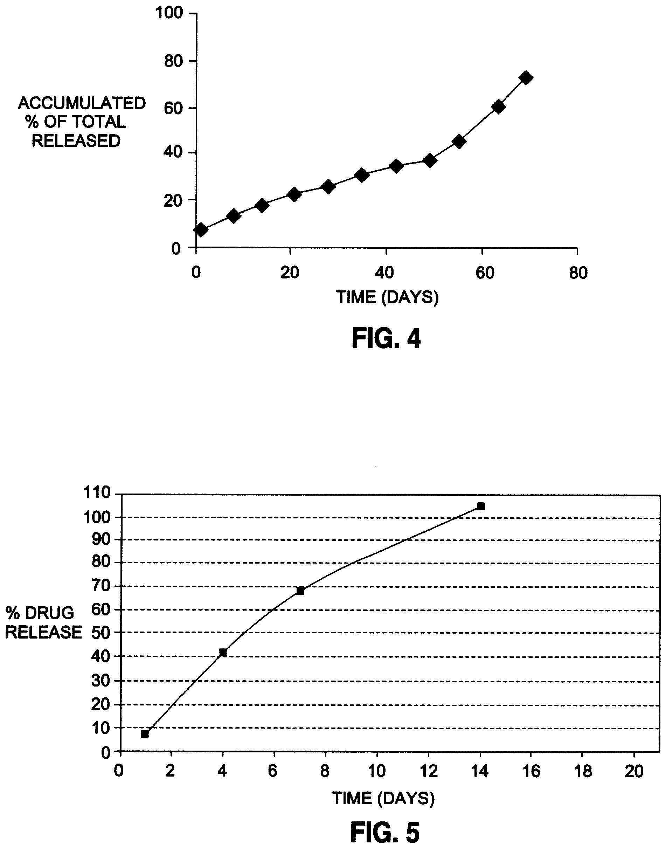

With these process improvements, Formulation A microspheres were made with the polymer 75:25 Poly(D,L, lactide-coglycolide)(Resomer RG755, Boehringer Ingelheim, Ingelheim, Germany) with a latanoprost content of 23.8% (reference example). Latanoprost (200 mg), a viscous oil at room temperature, and the polymer (600 mg) were dissolved in 5.6 ml ethyl acetate. This solution was added to 160 ml 1% PVA water via a micro-pipette while shearing. The mixture at 3000 rpm for 5 min with a Silverson homogenizer. After shearing, the milky white emulsion was mildly agitated in a hood for 3-5 hrs to allow solvent evaporation. The suspension was passed through 106 µm and 34 µm sieves to remove any fractions bigger than 106 µm and smaller than 34 µm. The supernatant was removed by centrifuging the suspension
at 2000 rpm for 15 min, and 10 ml DI water was added to reconstitute the microspheres. The microsphere suspension was lyophilized to obtain free flowing dry powder. The vehicle used to suspend the microspheres before injection was 2% CMC and 0.1% wt Tween 80 (polysorbate 80) in 0.9% saline. The mean microsphere diameter size was about 60 µm. The *in vitro* release rate (from a 50 µl dose of 20% microspheres) in a PBS medium with 0.1% Triton X-100 [octylphenol polyethoxylate]) of the latanoprost from the Formulation A microspheres was with zero order (constant amount of drug released per unit time) release kinetics over a significant period of time, as shown by Figure 4. The Formulation A microspheres showed *in vitro* release rates of about 21 µg/day for the first 2 weeks.

Typically a sustained release drug delivery system releases incorporated drug following first order release kinetics, by which initial high levels of drug are released followed by a decrease (often an exponential decrease) in the drug release rate. Such a variable rate of drug dosing (over dose followed by under dose) to a target tissue is suboptimal for therapeutic treatment of an ocular condition. On the other hand first order drug release is optimal and is a highly beneficial dosing regime for successful treatment of intraocular tissues.

The microspheres were suspended in above mentioned vehicle at 20% concentration. The resulting suspension was injected through the scleral into the anterior chamber or into the vitreous through a 25G needle. Alternatively, the microspheres can be suspended in a variety viscous gels, and they can be injected with as small as a 30G needle. Two to ten milligrams microspheres were injected into dog eyes and saw significant IOP (50% from baseline) decrease for a period of time greater than 5 weeks. The microspheres can be injected into different sections of an eye including intracameral, intravitreal, and subtenon. The release rates can be adjusted by using different doses of the microspheres. The microspheres can also be injected with an applicator that allows for a 'dry' injection with or without the use of an aerosol. Here, the microspheres without the use of a wet vehicle can be injected into the anterior chamber or vitreous cavity without appreciably increasing the volume of the compartment. The Formulation A microsphere formulation has the potential to release latanoprost for between 2 to 7 months with a single intracameral or intravitreal injection.

### Formulation B Microspheres (reference)

Formulation B microspheres were made with Resomer R203H (poly-DL-lactic acid) ("PLA") with latanoprost content of 12.4% (reference example). The manufacturing process was similar to the Formulation A microsphere process set forth above. The microspheres were screened to isolate those with diameters equal to or greater then 34 microns and the resultant mean diameter was 45 microns. The in vitro release rates in PBS medium with 0.1 % Triton X-100 (octylphenol polyethoxylate) exhibited near zero order release kinetics are shown in Figure 5. The in vitro release rates of the Formulation B microspheres releasing was 88 µg/day over a 2 week period.

We discovered that carrying out a further processing step upon the microspheres made provided the important feature of reducing an undesirable side effect upon *in* vivo administration of the microspheres. Thus, one side effect of microspheres injected into an eye can be corneal hyperemia. We determined that use of the two purification steps of size fractionation and washing prevented almost all hyperemia upon intraocular injection of the so further processed microspheres. These two steps were carried out by filtering the microsphere suspension through 34 µm sieves to remove any smaller population of microspheres followed by washing the resulting microparticles were washed with water 3 to 5 times. When these purified microspheres were injected intracameral into dog eyes, there was significant (50 to 80% reduction) improvement in the corneal hyperemia observed.

### Summary

Microspheres were successfully manufactured with a solvent extraction process. Homogenization shear rates and polymer concentrations were found to be the main factors for particle size control. The microsphere diameters can be varied from 1 um to up to 100 µm, and fractionation with sieves produced well defined size ranges. Latanoprost loading can be optimized to 25 wt %. Microspheres can be lyophilized without any protectant and showed remarkable size stability. We found that e beam irradiation at moderate dosage (18 KGy) achieved excellent sterilization without any impact on subsequent drug release from the microspheres. A wide variety of release profiles were achieved mainly by using different polymer matrixes. The microspheres showed different morphology closely related to polymer properties and process conditions. Microsphere tends to settle fast and a high viscosity vehicle, for example 2% CMC, can slow down the settlement and make injection easier. Injections with 27 to 30G needles can be obtained upon use of a suitable gel carrier.

Examples 3 to 7 below set forth *in vivo* (Beagle dogs) studies carried out by intraocular injection of microspheres or implant, suspended in aqueous vehicle (2% carboxy methylcellulose [CMC], 0.1 % Tween 80 in 0.9% saline) or in a high viscosity, high shear rate gel (i.e. a suitable high molecular weight, polymeric hyaluronic acid). Microspheres or implant containing an anti-hypertensive agent was injected in one eye and placebo microspheres or implant was injected in the other eye as a control. IOP and hyperemia were monitored for multiweek periods. Ultra thin wall 25-27 gauge needles were used. When microspheres were used they were suspended in vehicle at 10% or 20% by weight.

### Example 3 (reference)

### Intracameral Formulation A Microspheres

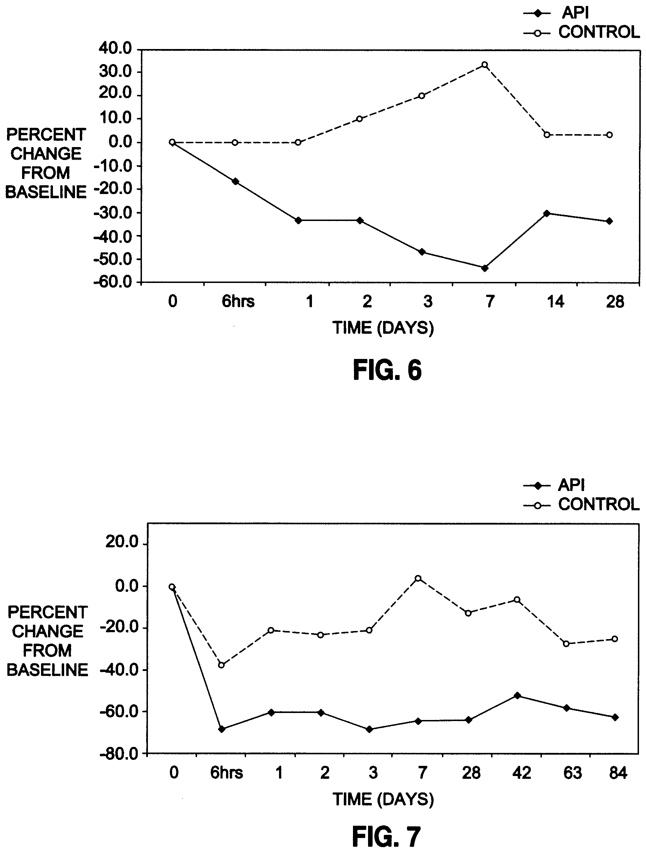

10 ul of Formulation A was injected into the left anterior chamber of a beagle dog using a shelving approach through the cornea with a 25G hypodermic needle at the 12 o'clock position. The wound was self-sealing and the microspheres rapidly settled into the inferior angle within 30 minutes. Figure 6 demonstrates a profound reduction in IOP in the left eye compared with the untreated right eye. This reduction in IOP was sustained for a number of weeks. As shown by Figure 6, the left eye (solid line) received an intracameral injection of sustained-release latanoprost microspheres and IOP reduction of approximately 50% from baseline was recorded in the left eye by day 3 and an IOP reduction was sustained to at least the 1 month time point. No reduction of IOP was noted in the fellow control eye (dashed line) that received no injection. External photographs of the eye show only mild conjunctival hyperemia.

### Example 4 (reference)

### Intracameral Formulation B Microspheres

A dog received an intravitreal injection anteriorly with 50 µl of the Formulation B microsphere formulation in the left eye 4 mm behind the limbus in the supero-nasal quadrant, the right eye received an injection of placebo microspheres. In the left eye, the IOP was reduced to a maximum of approximately 40% below baseline and there was mild to moderate conjunctival hyperemia localized to the site of injection. The hyperemia grades in the opposite quadrants were consistently recorded as 0. There was no IOP reduction seen in the right eye that received the placebo microspheres.

### Example 5 (reference)

### Intravitreal and Intracameral Bimatoprost Implant

Sustained-release bimatoprost heat extruded implants were made using 45 wt% resomer R203s (poly-DL-lactic acid), 20 wt% R202H (Poly (DL- lactide)), 30 wt% drug load, and 5wt% PEG3350 as cosolvent. The total weight of the bar-shaped implants made were either 1.64mg (492.4ug drug load) or 800 µg, the latter, half size bar shaped implant measuring 1 mm wide and 2 mm long. The implants showed in vitro release of bimatoprost a over a four month period.

### Intravitreal

One bimatoprost implant was inserted in the supero-nasal quadrant of the left dog eye anterior vitreous, 4 mm behind the surgical limbus. A placebo (no bimatoprost) implant was placed in the fellow eye. There was a significant reduction of IOP (up to 45% below baseline) in the left vs. right eye). In addition, there was considerably less conjunctival hyperemia with intravitreal implant placement compared with sub-Tenon's placement.

### Intracameral

One 800 µg bimatoprost implant was inserted into the anterior chamber through a shelved incision in clear cornea superiorly near the limbus of the right eye, the left eye received a placebo (no bimatoprost) implant. The implant was located superiorly at the 6 hour time point and settled inferiorly at the 6 o'clock position by 24 hours after insertion. There was slow bioerosion of the implant noted and no signs of intraocular toxicity. There was a large reduction in the IOP ranging from 60 to 70% below baseline recordings noted in the first 24 hours and maintained thereafter. See Figure 7.

The implant released about 6 µg bimatoprost each day for the first 30 days after administration. The implant upon administration fit well into the well of the angle along the trabecular meshwork at the 6 o'clock position. The anterior chamber angle exists where the cornea meets the iris. At this location one finds the trabecular meshwork which is the site where (in a normal eye) the aqueous humor drains out of the eye. If the aqueous humor cannot properly drain out of the eye, elevated intraocular pressure results. Figure 7 is a graph of percent change from subject dog eye baseline intraocular pressure (y axis) against time in days (x axis) over the 84 day period after intracameral administration of the bimatoprost bar shaped implant, showing that an IOP drop of 50% to 60% was maintained through the 84 day observation periods, showing great superiority of this single intracameral sustained release implant over the alternative daily (at least once each day for 84 days) anti-hypertensive agent eye drop administration to treat elevated IOP.

### Example 6

### Intracameral and Intravitreal EP2

The safety and tolerability of a single intracameral and intravitreal neat injection of the EP2 agonist Compound A (a molecule with a chiral center), was evaluated. The formulation used 0.1 % of Compound A in normal saline. The chemical name of Compound A is 5-{(R)-1-[4-((S)-1-Hydroxy-hexyl)-phenyl]-5-oxo-pyrrolidin-2-ylmethoxymethyl}-thiophene-2-carboxylic acid isopropyl ester, its chemical formula is C₂₆H₃₅NO₅S and its molecular weight is 473.63.

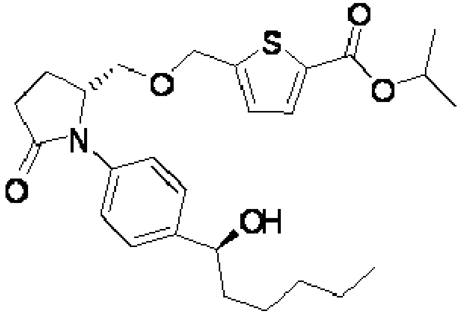

### Dog 1: Intracameral Injection

A 50 microliter injection of Compound A formulation was performed into the anterior chamber of the left eye, the vehicle in the right eye, using a 27G hypodermic needle. The IOP was reduced to a maximum of approximately 50% from baseline in the right eye. IOP reduction was maintained through day 3 compared to the fellow eye recordings. There was a maximum conjunctival hyperemia score of +0.5 present near the site of injection of both eyes in the first day, and subsequent recordings were all 0. There were no signs of ocular inflammation at the follow up examinations.

### Dog 2: Anterior Vitreous Injection

A 50 microliter injection of the Compound A formulation was performed in the anterior vitreous of the left eye, entering just posterior to the ciliary body using a 27G hypodermic needle. Vehicle was injected into the right eye. The IOP was reduced to a maximum of approximately 30% from baseline in the right eye. IOP reduction was maintained through day 3 compared to the fellow eye recordings. There was mild conjunctival hyperemia through day 3 in both eyes localized to the hemisphere where the injection occurred. There were no signs of ocular inflammation at the follow up examinations.

In summary, neat injections of Compound A in the anterior chamber and anterior vitreous were well tolerated and there was lowering of the IOP for a number of days following a single injection. In addition to reducing IOP an EP2 and EP4 agonist are can also be potent neuroprotective agent.

Compound A can be formulated in biodegradable polymeric microspheres or in a biodegradable polymeric implant, using the methods set forth in the Examples above, and administered intracameral or into the anterior vitreous to provide sustained anti-hypertensive glaucoma treatment effect.

### Example 7 (reference)

### Intracameral Latanoprost Implants

A sustained-release latanoprost (heat extrusion) implant comprising 30% latanoprost, 40% RG752s, 20% RG502s, 5% Plasdone and 5% PEG 3350 was made and intracameral injected into the left eye of a dog. A shelving incision was performed at the 11 o'clock position with a keratome and the latanoprost implant was inserted into the anterior chamber. The incision was closed using a 9-0 vicryl suture. There was approximately a 50% reduction of IOP from baseline recorded in this eye at the 24 hour time point.

### Example 8 (reference)

### Formulation A Microspheres in Hyaluronic Acid

Formulation A microspheres with a drug content of 23.8% were mixed with a cross-linked hyaluronic acid (Juvederm). Using a gel-based microsphere suspension, a 27G needle was used to inject the left eye with the active microsphere, the right eye with the placebo. The injection was facilitated by using the gel and there were no stoppages due to the needle becoming clogged. On day 1 post-injection, there was a 35% reduction in IOP in the left eye compared with baseline values. The microspheres appeared aggregated in the gel in the inferior angle and there was minimal ocular inflammation.

### Example 9

### Anti-Hypertensive Drug Containing Microspheres and Implants

Anti-hypertensive drug containing sustained release, biodegradable microspheres can be made for intracameral or anterior intravitreal injection to treat a hypertensive condition such as glaucoma. The anti-hypertensive drug can be one or more of EP2 agonists, such as Compounds A to O, and their salts and esters. As noted below each of Compounds A to H and J has at least one chiral center. The microspheres can be made with the polymer 75:25 Poly(D,L, lactide-coglycolide)(Resomer RG755, Boehringer Ingelheim, Ingelheim, Germany) with a Compound (any of Compounds A to O) weight % content between 15 to 25 wt %. Thus 200 mg of a Compound (any of Compounds A to O) and 600 mg of the polymer are dissolved in about 6 ml ethyl acetate. This solution is then added to 160 ml 1% PVA water via a micro-pipette while shearing. The mixture is then centrifuged at about 3000 rpm for 5 min with a Silverson homogenizer. After shearing, a milky white emulsion can be obtained which is mildly agitated in a hood for 3 to 5 hrs to allow solvent evaporation. The suspension is then passed through 106 µm and 34 µm sieves to remove any fractions bigger than 106 µm and smaller than 34 µm. The supernatant is removed by centrifuging the suspension at 2000 rpm for 15 min, and 10 ml DI water is added to reconstitute the microspheres. The microsphere suspension is then lyophilized to obtain a free flowing dry powder. The vehicle used to suspend the microspheres before injection can be 2% CMC and 0.1% wt Tween 80 (polysorbate 80) in 0.9% saline.

Additionally, anti-hypertensive drug containing, sustained release, biodegradable implants can be made for intracameral or anterior intravitreal injection to treat a hypertensive condition such as glaucoma. The anti-hypertensive drug can be one or more of EP2 agonists, such as Compounds A to O. The implants can be made by hot-melt extrusion to contain 30 wt% Compound (any of Compounds A to O), 40-60 wt% of a biodegradable poly (D,L-lactide-co-glycolide) polymer (Resomer® RG752s)(PLGA), 0-20% of a biodegradable poly (D,L-lactide) polymer (Resomer® R202s)(PLA), and 10% PEG-3350.

The Compound containing bioerodible polymer implants in this Example can be made by hot-melt extrusion using a mechanically driven ram microextruder but they can also be made by direct compression or solvent casting. The implants are preferably rod-shaped, but they can be made into any geometric shape by changing the extrusion or compression die. Polymers (the resomers) are used as received from Boehringer Ingelheim.

Compound (any of Compounds A to O) and polymer resomer powder are initially mixed (including 10 weight% PEG) using a spatula in a weigh-boat for 15 minutes. The mixture is then transferred into a stainless steel container containing two ¼" stainless steel ball and mixing is continued using a Turbula mixer for two separate 15 minute cycles. The powder blend is mixed by hand using a spatula between each cycle and after the final cycle. The blended material is then compacted into an extruder barrel and the extruder barrel is placed into the heated well (between 50 and 55 degrees C.) of the piston extruder and extruded using 500 pm nozzle and a speed setting number of 0.0025. The extruded filament (rod shaped) implants are cut into one milligram implant (approximately 3 mm long). These sustained release, biodegradable implants can be administered intracameral to provide from 1 to 6 months (or longer) reduced IOP (anti-hypertensive effect).

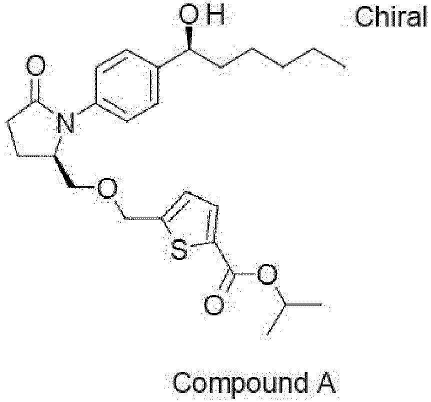

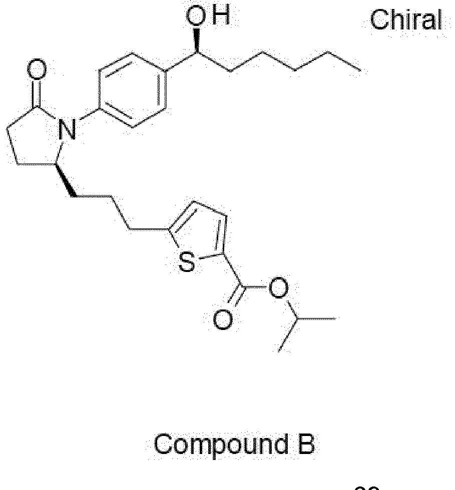

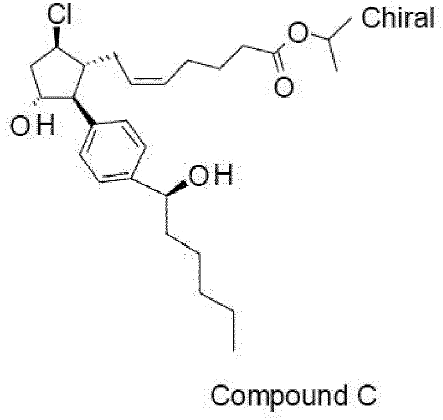

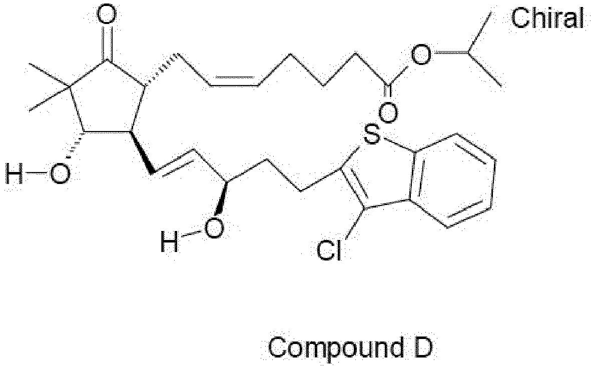

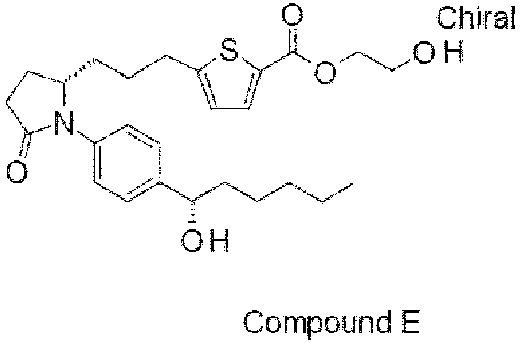

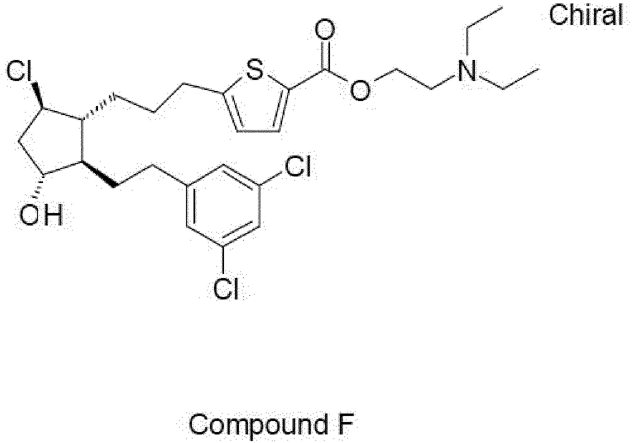

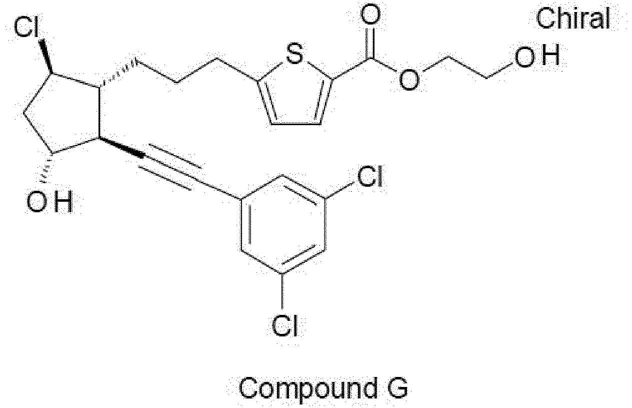

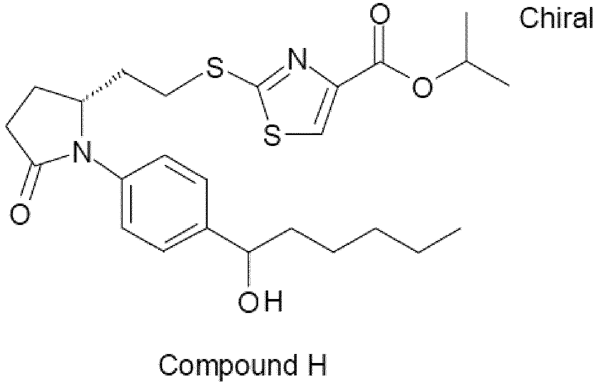

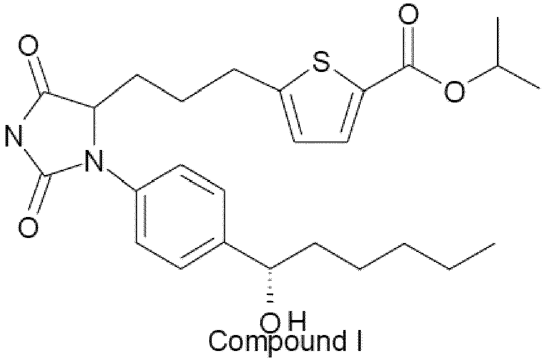

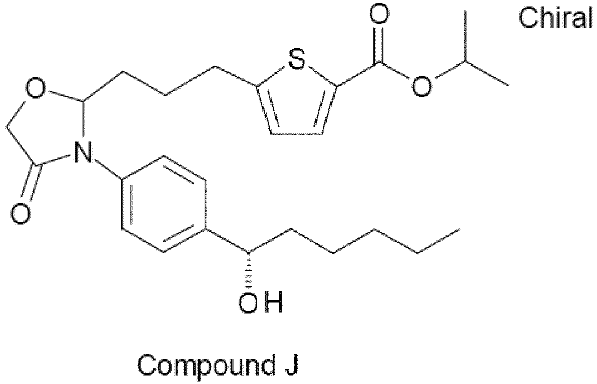

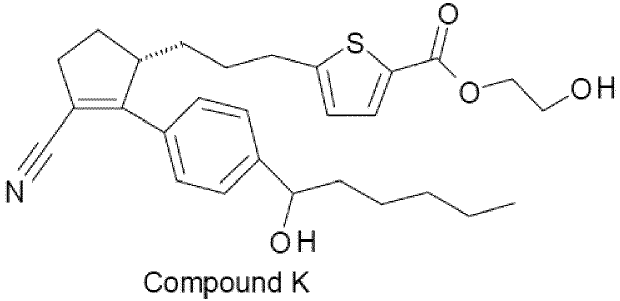

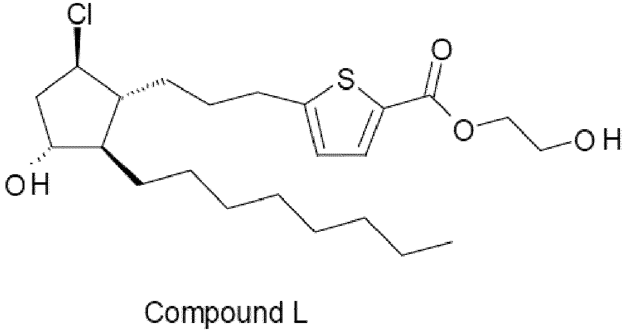

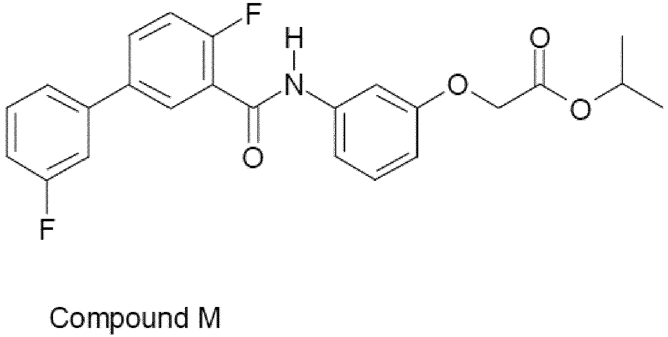

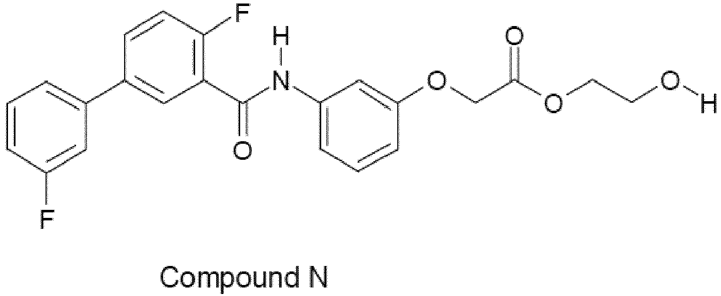

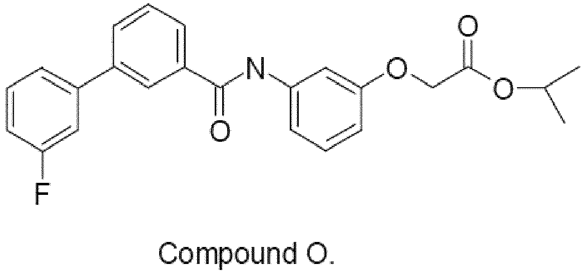

## Claims

1. A sustained release implant comprising an anti-hypertensive agent and a biodegradable polymer for use in a method for treating elevated intraocular pressure, the method comprising the step of intracameral or anterior vitreal administration, to a patient with elevated intraocular pressure (IOP), of said sustained release implant,
wherein the sustained release implant comprises from 10 to 50 weight percent of an anti-hypertensive agent and from 50 to 90 weight percent of a biodegradable polymer,
wherein the implant releases therapeutically effective amounts of the anti-hypertensive for a period of time between 10 days and 120 days, and
wherein the anti-hypertensive agent is selected from the group consisting of Compounds A to O, and their salts and esters:

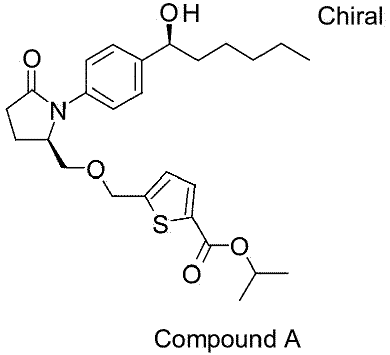

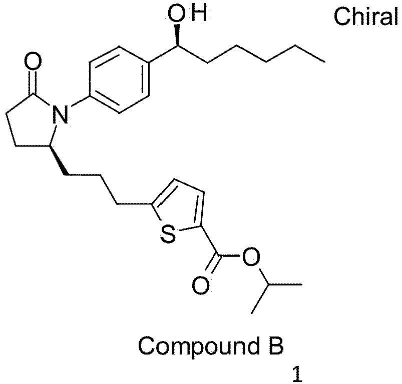

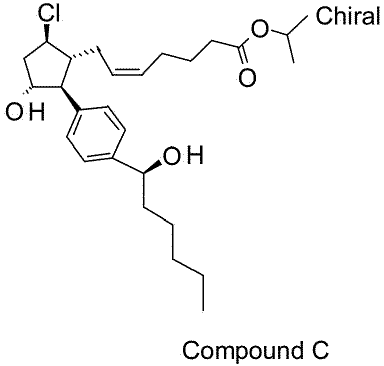

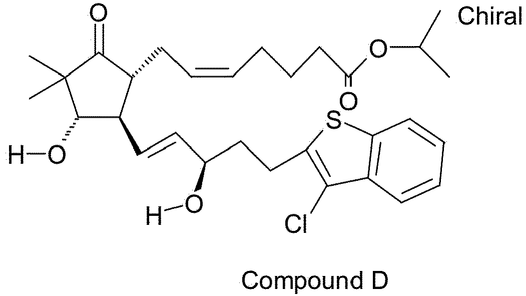

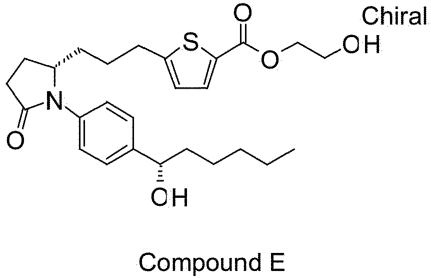

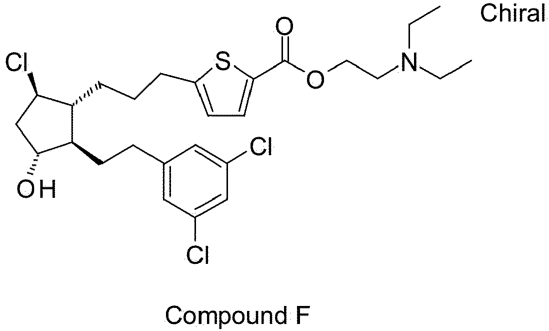

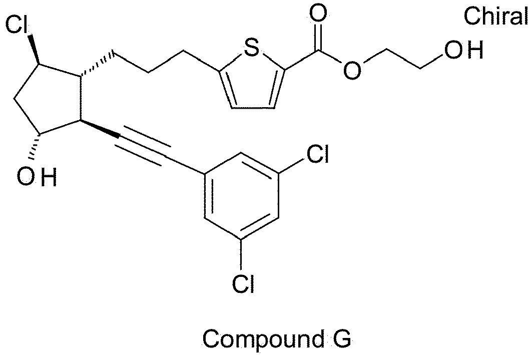

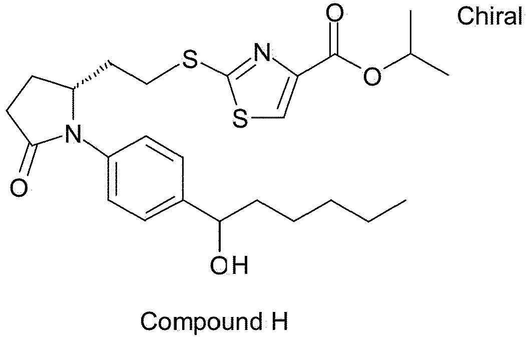

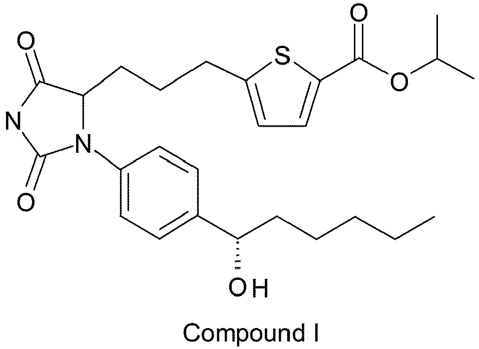

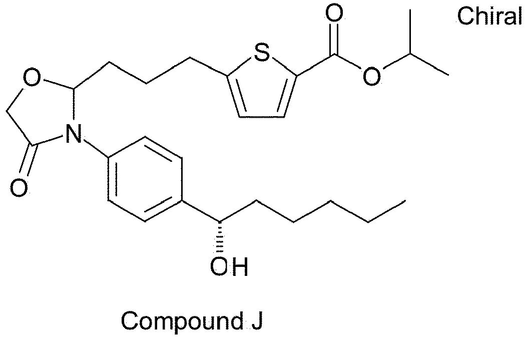

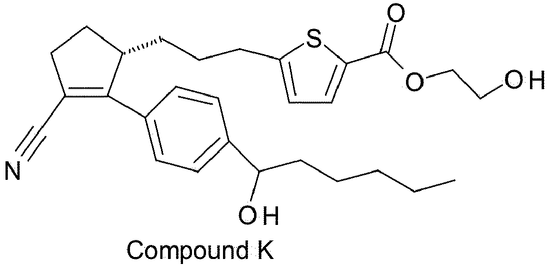

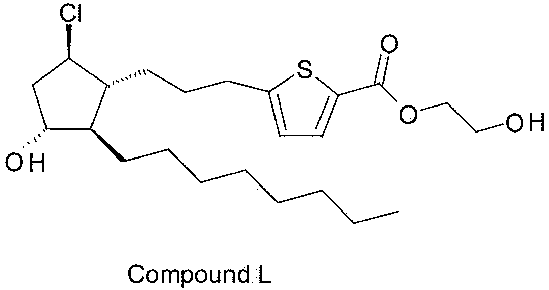

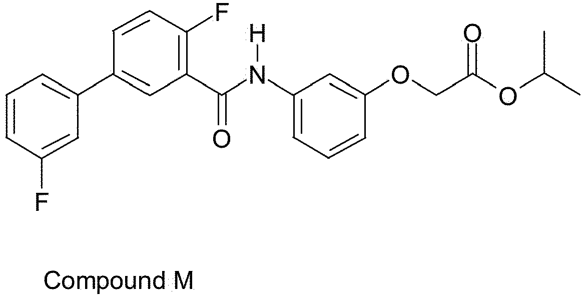

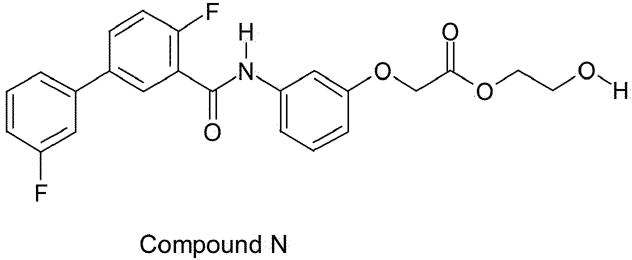

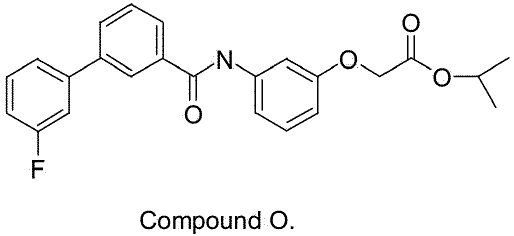

2. A plurality of sustained release biodegradable microspheres comprising an anti-hypertensive agent and a biodegradable polymer for use in a method for treating elevated intraocular pressure, the method comprising the step of intracameral or anterior vitreal administration to a patient with elevated intraocular pressure of said plurality of sustained release biodegradable microspheres having an average diameter between 30 and 60 microns,
the microspheres comprising from 10 to 30 weight percent of an anti-hypertensive agent and from 70 to 90 weight percent of a biodegradable polymer,
wherein the microspheres release therapeutically effective amounts of the anti-hypertensive agent for a period of time between 10 days and 120 days, and
wherein the anti-hypertensive agent is selected from the group consisting of Compounds A to O, and their salts and esters:

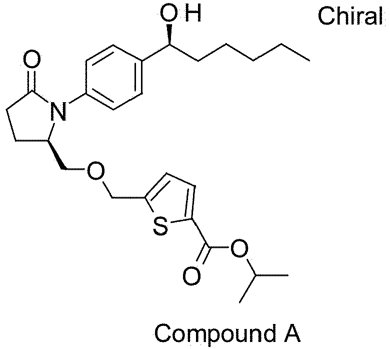

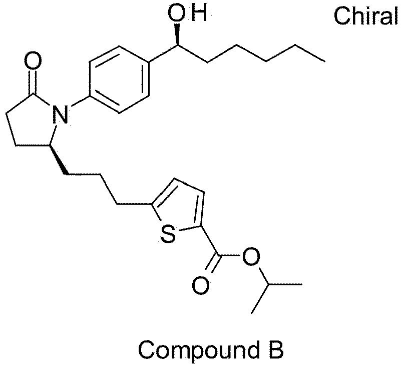

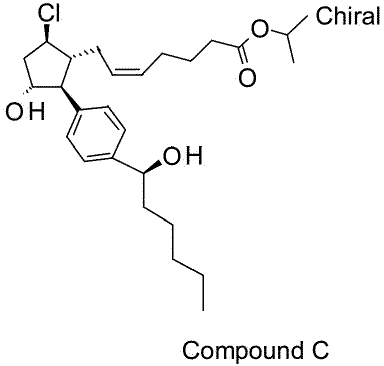

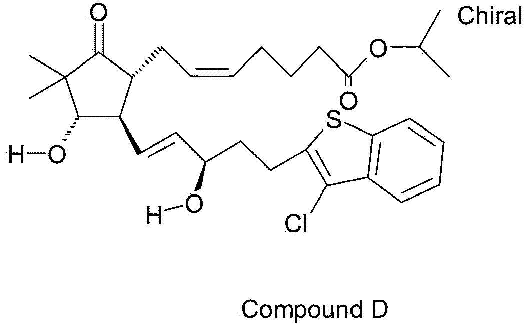

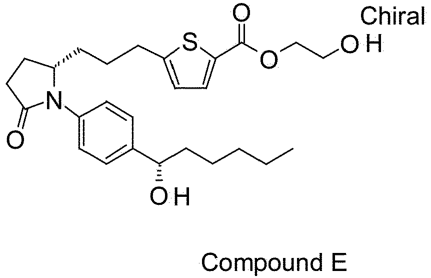

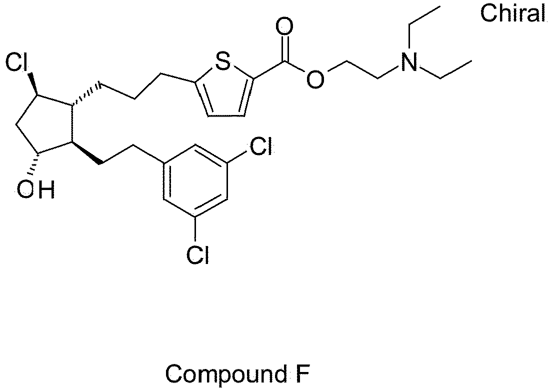

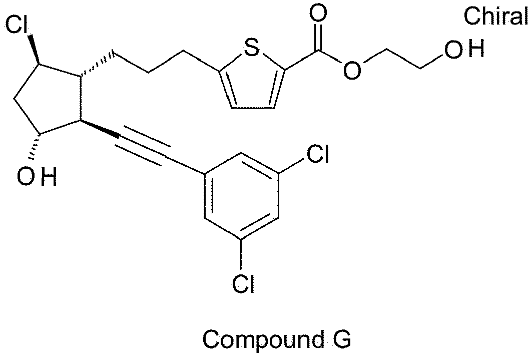

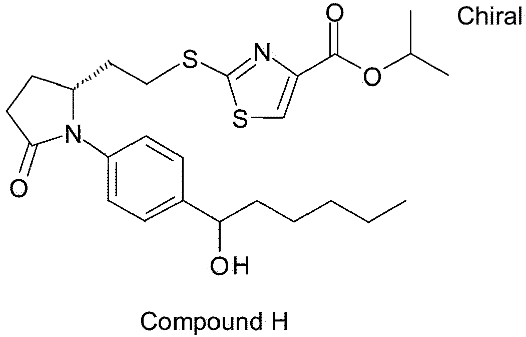

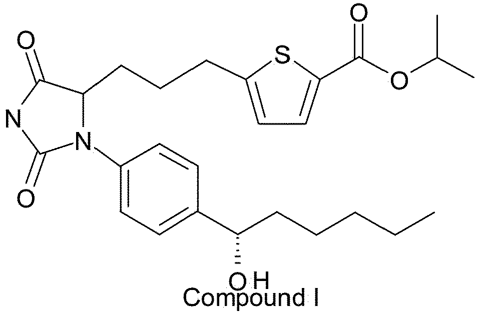

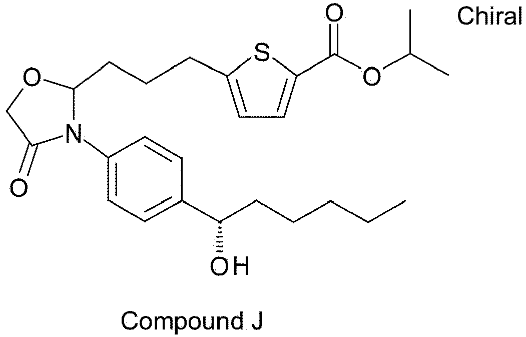

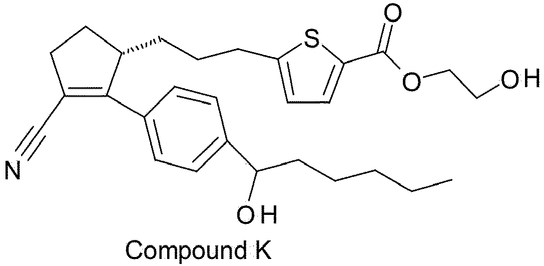

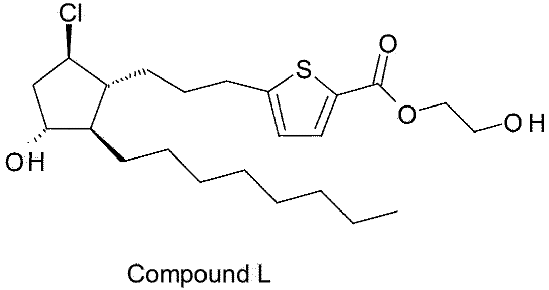

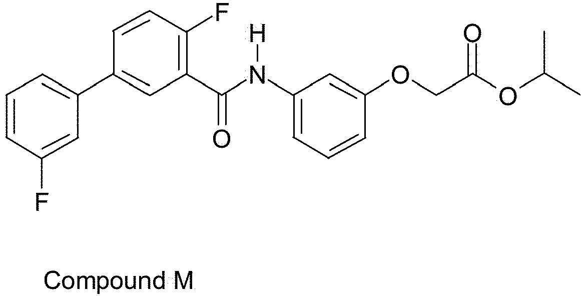

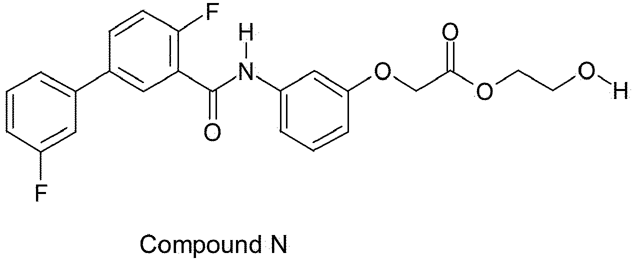

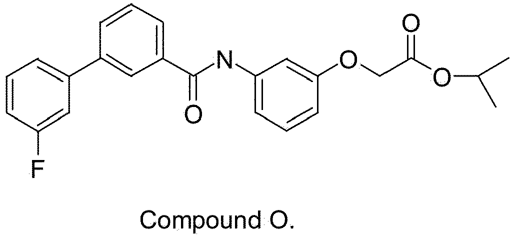

3. The microspheres for use of claim 2, wherein the biodegradable polymer comprises a polylactic polyglycolic copolymer (PLGA) and/or a poly lactic acid polymer (PLA).

4. The microspheres for use of claim 2, wherein the microspheres comprise a high viscosity hyaluronic acid with an average molecular weight between 1 and 4 million Daltons.

## Patentansprüche

1. Implantat mit verzögerter Freisetzung, das ein blutdrucksenkendes Mittel und ein biologisch abbaubares Polymer umfasst, zur Verwendung in einem Verfahren zur Behandlung von erhöhtem Augeninnendruck, wobei das Verfahren den Schritt der intrakameren oder anterioren vitrealen Verabreichung des Implantats mit verzögerter Freisetzung an einen Patienten mit erhöhtem Augeninnendruck (IOP) umfasst,
wobei das Implantat mit verzögerter Freisetzung 10 bis 50 Gewichtsprozent eines blutdrucksenkenden Mittels und 50 bis 90 Gewichtsprozent eines biologisch abbaubaren Polymers umfasst,
wobei das Implantat therapeutisch wirksame Mengen des blutdrucksenkenden Mittels über einen Zeitraum zwischen 10 Tagen und 120 Tagen freisetzt, und
wobei das blutdrucksenkende Mittel aus der Gruppe ausgewählt ist, die aus den Verbindungen A bis O und deren Salzen und Estern besteht:

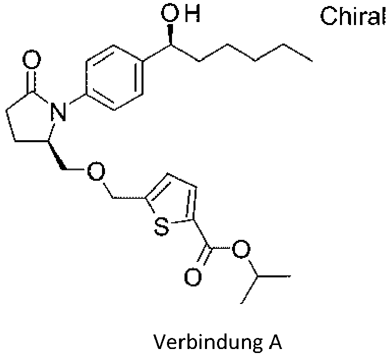

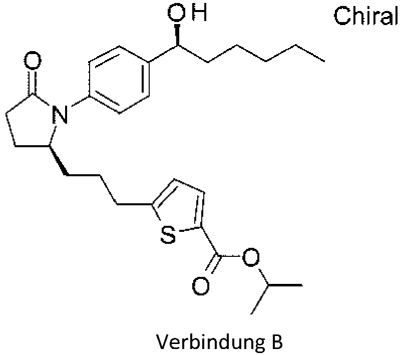

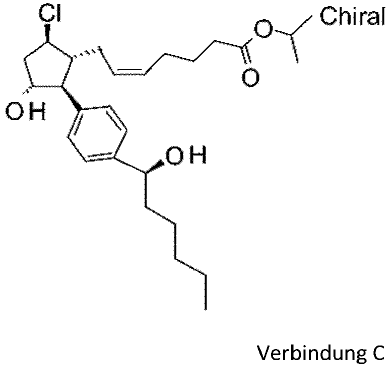

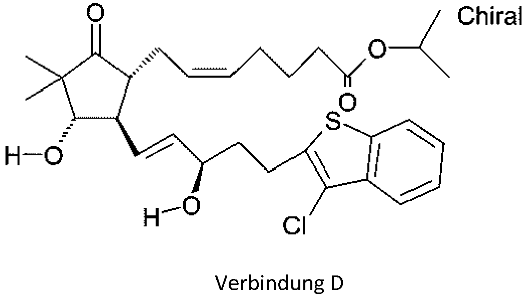

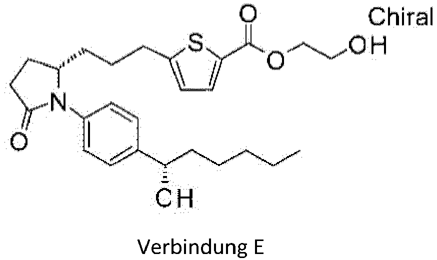

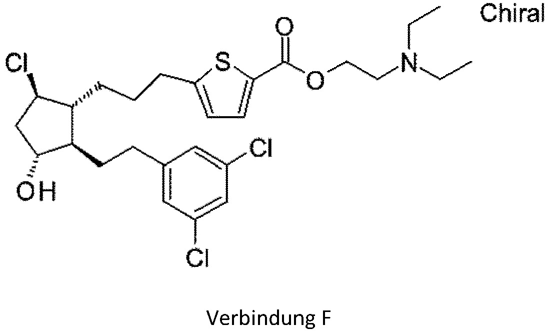

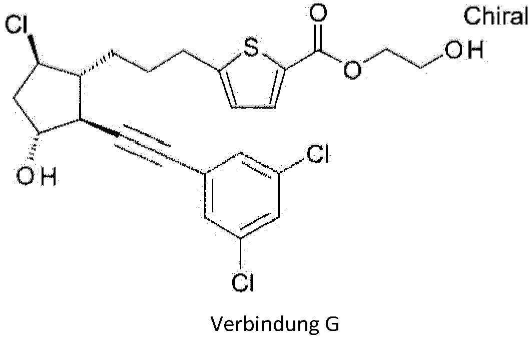

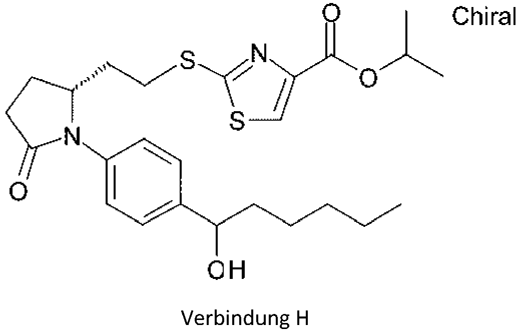

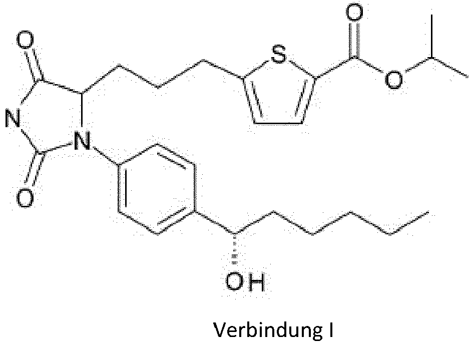

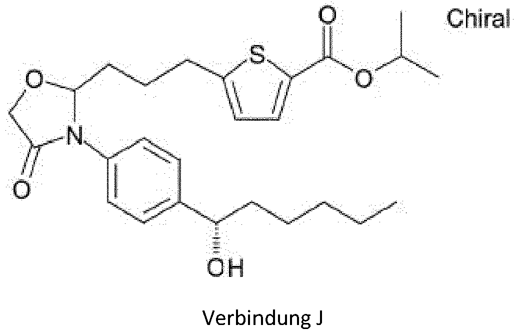

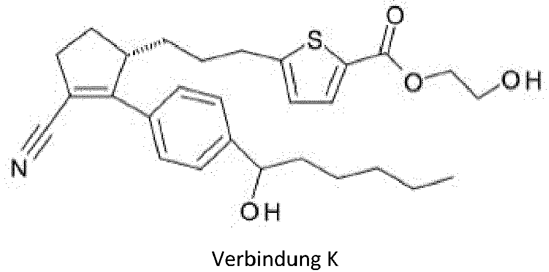

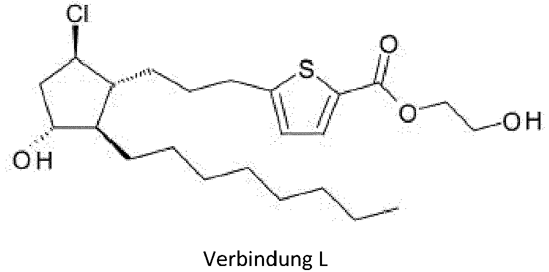

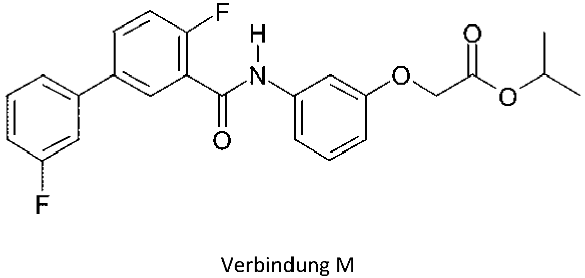

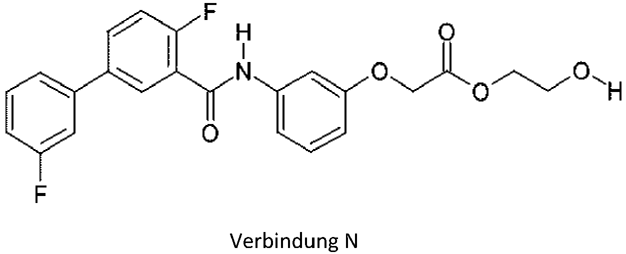

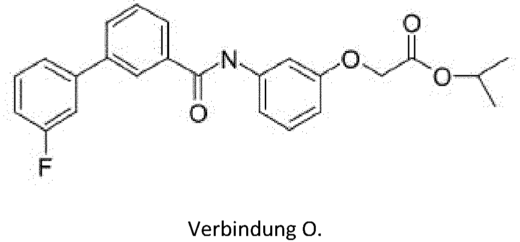

2. Vielzahl von biologisch abbaubaren Mikrosphären mit verzögerter Freisetzung, die ein blutdrucksenkendes Mittel und ein biologisch abbaubares Polymer umfassen, zur Verwendung in einem Verfahren zur Behandlung von erhöhtem Augeninnendruck, wobei das Verfahren den Schritt der intrakameren oder anterioren vitrealen Verabreichung der Vielzahl von biologisch abbaubaren Mikrosphären mit verzögerter Freisetzung an einen Patienten mit erhöhtem Augeninnendruck umfasst, die einen durchschnittlichen Durchmesser zwischen 30 und 60 Mikrometern aufweisen,
wobei die Mikrosphären 10 bis 30 Gewichtsprozent eines blutdrucksenkenden Mittels und 70 bis 90 Gewichtsprozent eines biologisch abbaubaren Polymers umfassen,
wobei die Mikrosphären therapeutisch wirksame Mengen des blutdrucksenkenden Mittels über einen Zeitraum zwischen 10 Tagen und 120 Tagen freisetzen, und
wobei das blutdrucksenkende Mittel aus der Gruppe ausgewählt ist, die aus den Verbindungen A bis O und deren Salzen und Estern besteht:

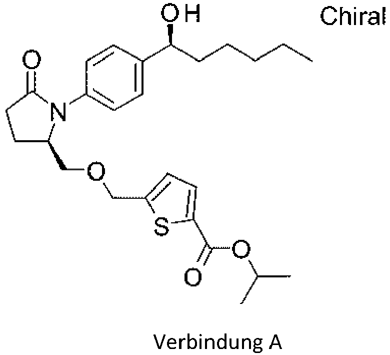

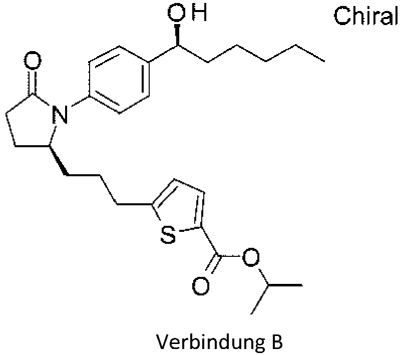

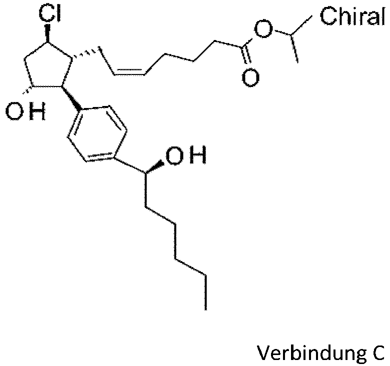

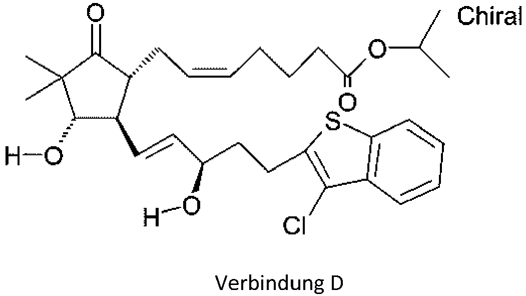

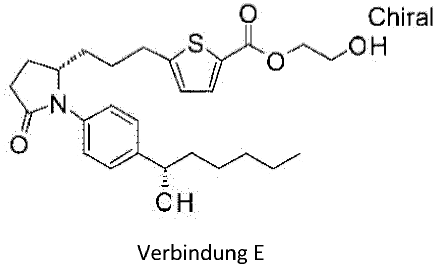

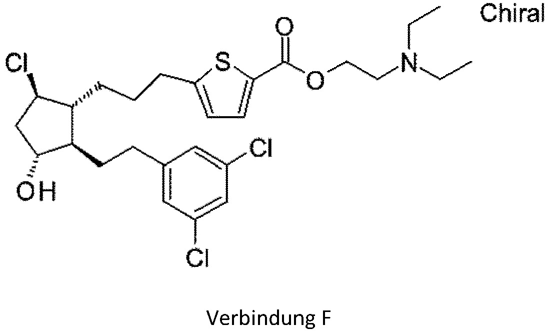

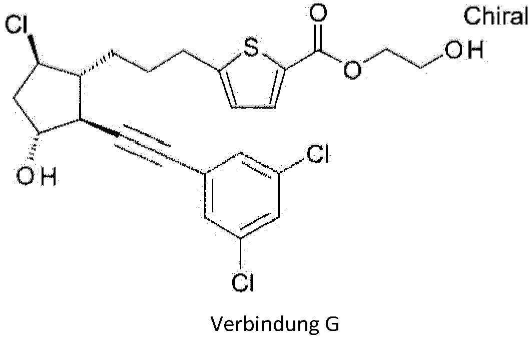

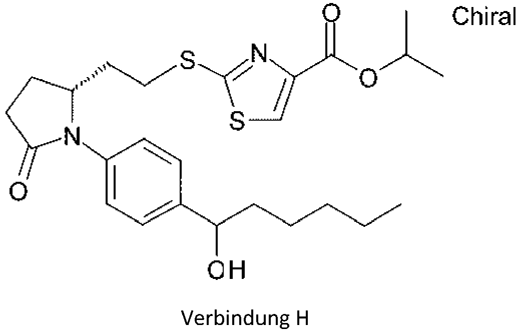

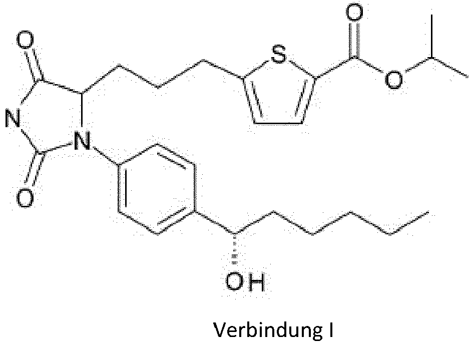

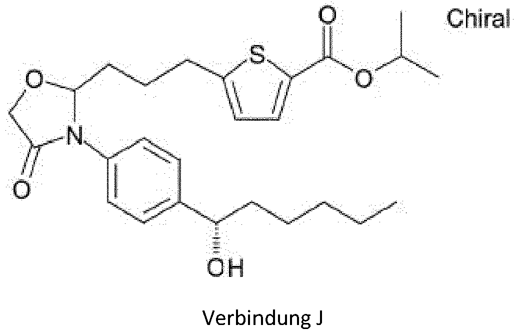

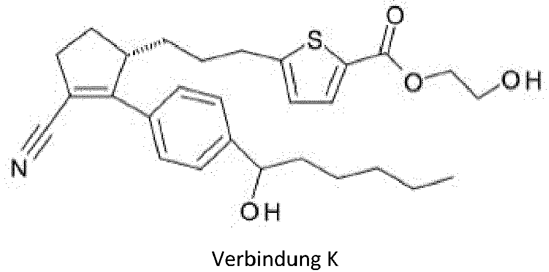

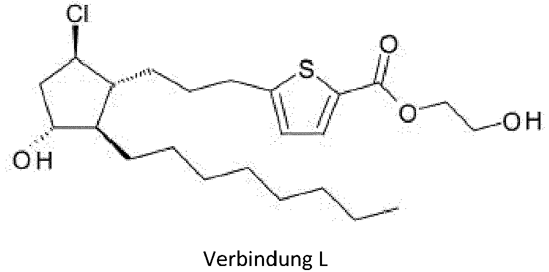

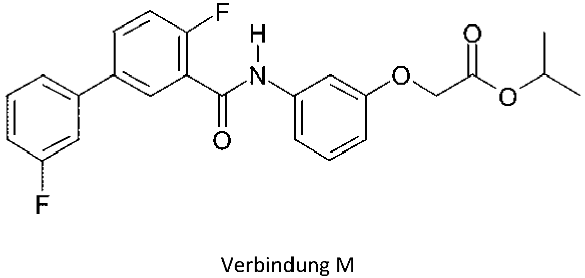

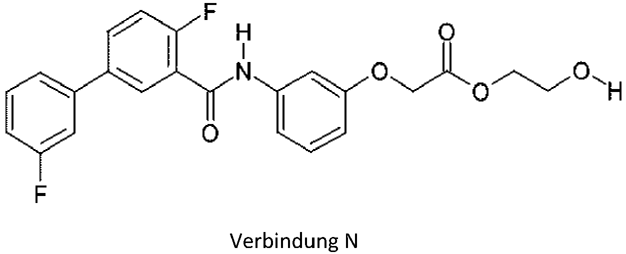

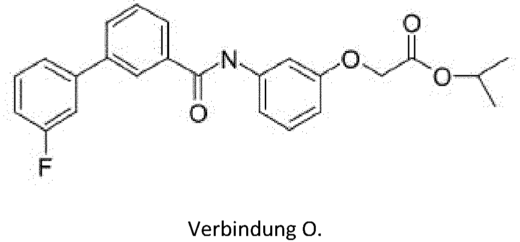

3. Mikrosphären zur Verwendung nach Anspruch 2, wobei das biologisch abbaubare Polymer ein Polymilchsäure-Polyglykol-Copolymer (PLGA) und/oder ein Polymilchsäure-Polymer (PLA) umfasst.

4. Mikrosphären zur Verwendung nach Anspruch 2, wobei die Mikrosphären eine hochviskose Hyaluronsäure mit einem durchschnittlichen Molekulargewicht zwischen 1 und 4 Millionen Dalton umfassen.

## Revendications

1. Implant à libération prolongée comprenant un agent antihypertenseur et un polymère biodégradable pour utilisation dans un procédé de traitement d'une pression intraoculaire élevée, le procédé comprenant l'étape d'administration vitréenne intracamérulaire ou antérieure, à un patient de pression intraoculaire (IOP) élevée, dudit implant à libération prolongée,
dans lequel l'implant à libération prolongée comprend de 10 à 50 pour cent en poids d'un agent antihypertenseur et de 50 à 90 pour cent en poids d'un polymère biodégradable,
dans lequel l'implant libère des quantités thérapeutiquement efficaces de l'antihypertenseur pendant une période entre 10 jours et 120 jours, et
dans lequel l'agent antihypertenseur est choisi dans le groupe consistant en les composés A à O, et leurs sels et esters :

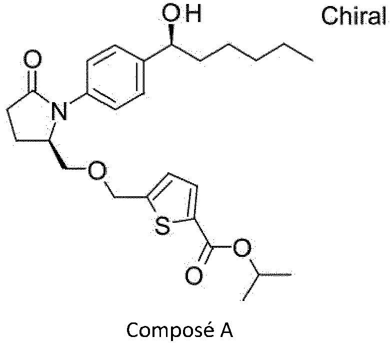

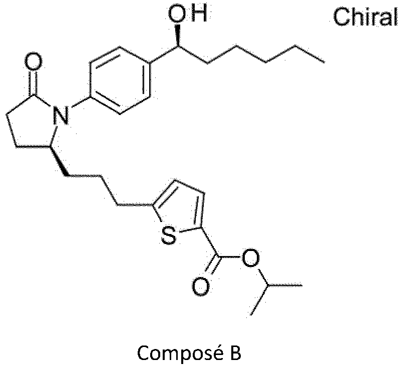

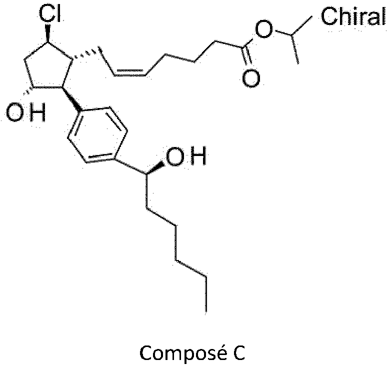

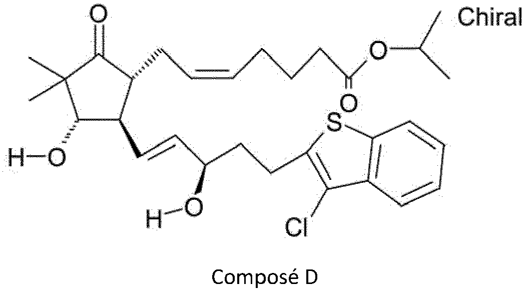

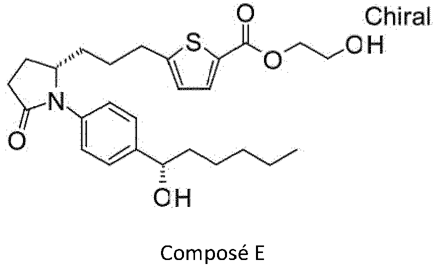

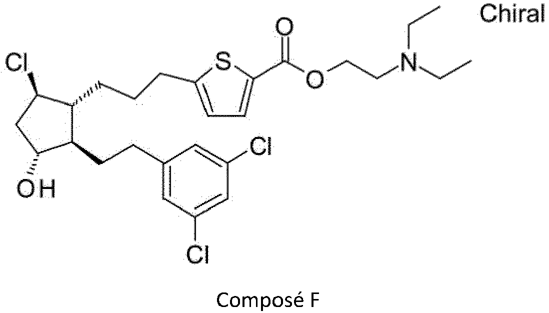

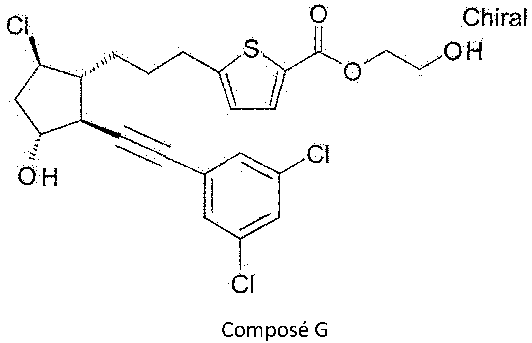

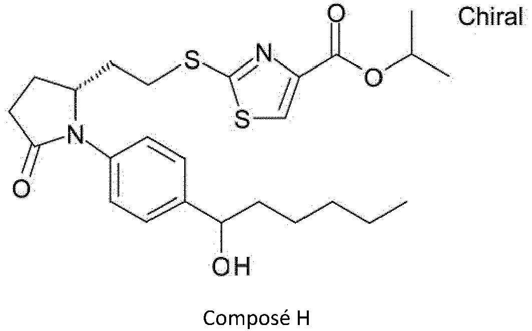

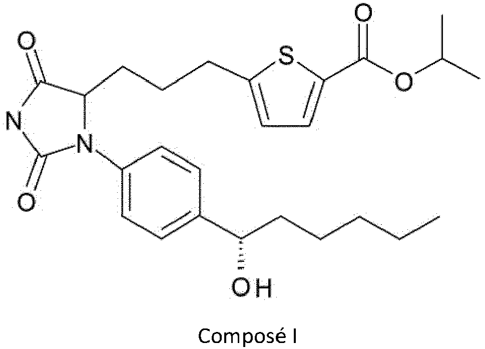

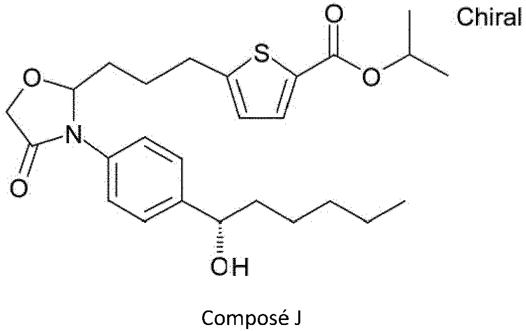

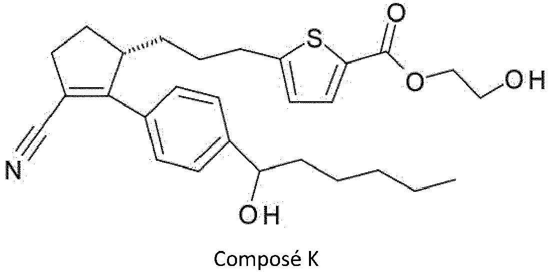

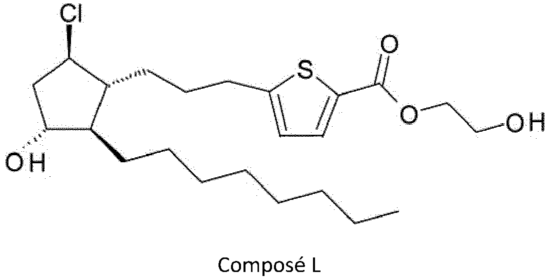

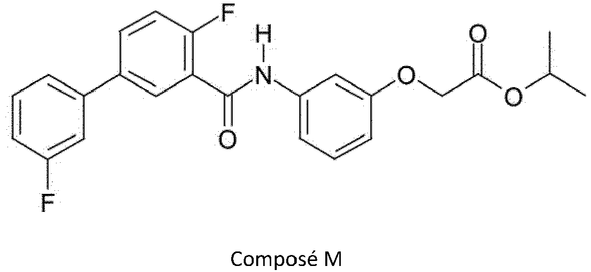

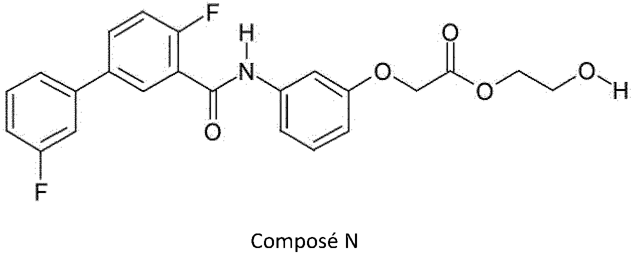

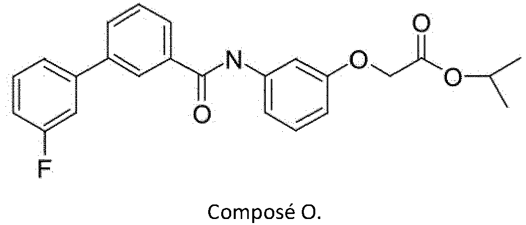

2. Pluralité de microsphères biodégradables à libération prolongée comprenant un agent antihypertenseur et un polymère biodégradable pour utilisation dans un procédé de traitement d'une pression intraoculaire élevée, le procédé comprenant l'étape d'administration vitréenne intracamérulaire ou antérieure à un patient de pression intraoculaire élevée de ladite pluralité de microsphères biodégradables à libération prolongée ayant un diamètre moyen entre 30 et 60 microns,
les microsphères comprenant de 10 à 30 pour cent en poids d'un agent antihypertenseur et de 70 à 90 pour cent en poids d'un polymère biodégradable,
dans laquelle les microsphères libèrent des quantités thérapeutiquement efficaces de l'agent antihypertenseur pendant une période entre 10 jours et 120 jours, et
dans laquelle l'agent antihypertenseur est choisi dans le groupe consistant en les composés A à O, et leurs sels et esters :

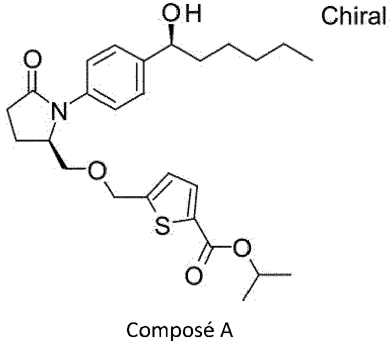

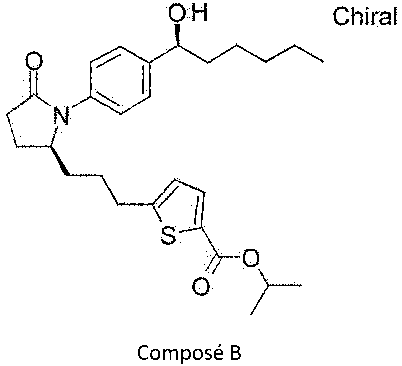

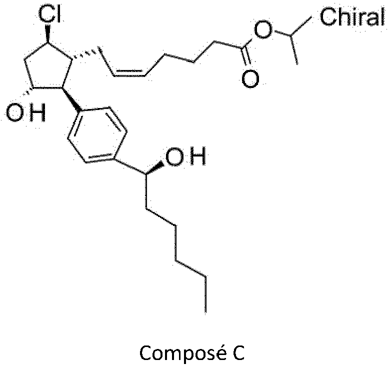

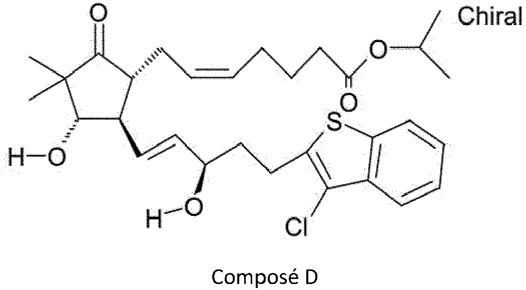

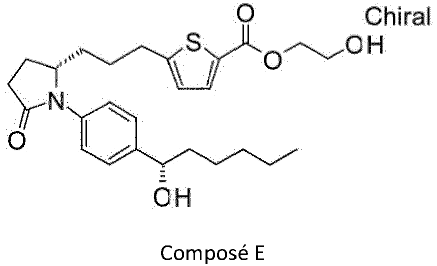

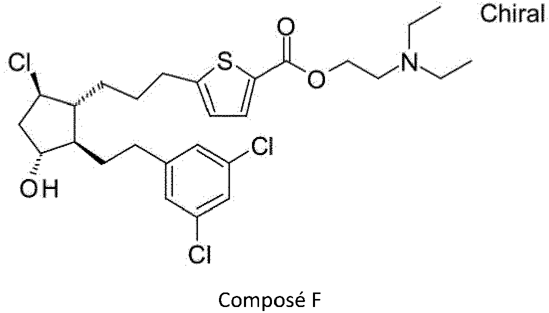

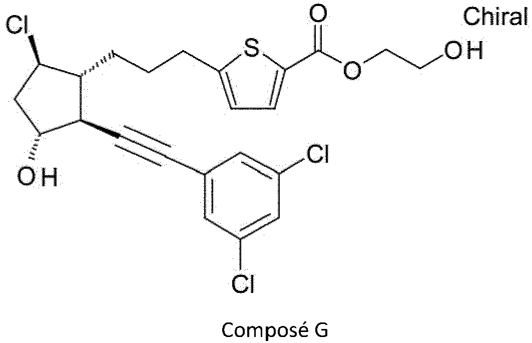

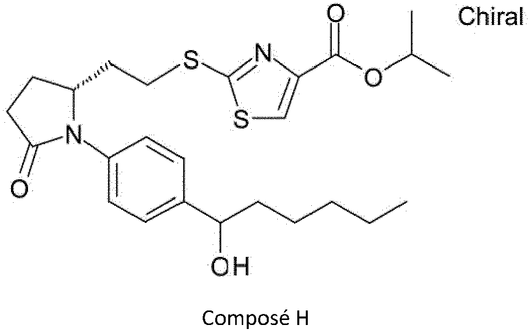

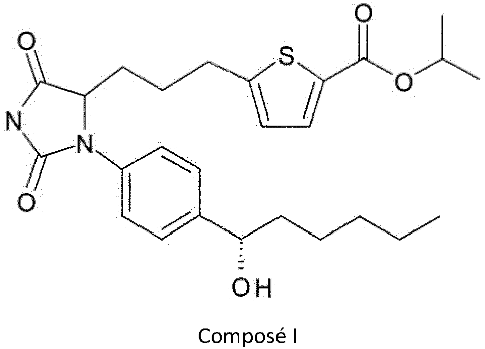

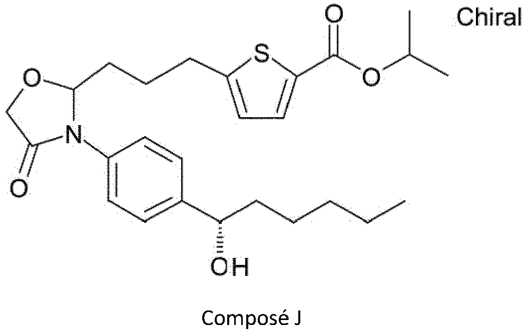

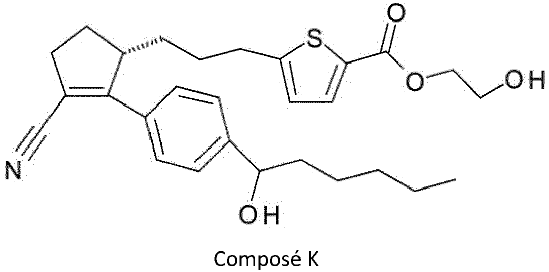

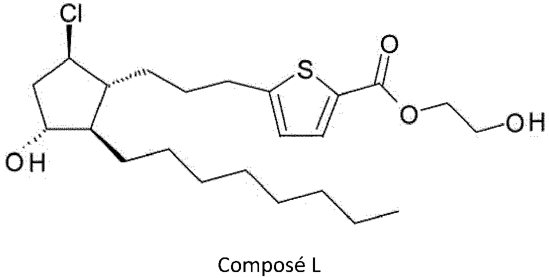

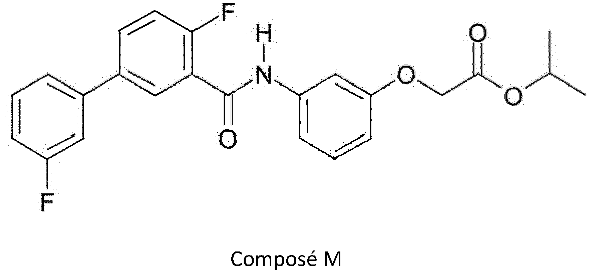

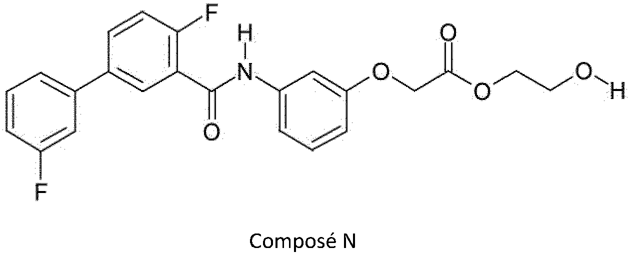

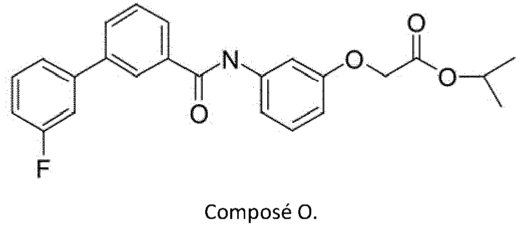

3. Microsphères pour utilisation selon la revendication 2, dans lesquelles le polymère biodégradable comprend un copolymère polylactique polyglycolique (PLGA) et/ou un polymère poly (acide lactique) (PLA).

4. Microsphères pour utilisation selon la revendication 2, dans lesquelles les microsphères comprennent un acide hyaluronique de haute viscosité de masse moléculaire moyenne entre 1 et 4 millions de Daltons.
